# EUROPEAN PATENT SPECIFICATION

(11) **EP 3 508 221 B1**
(45) Date of publication and mention of the grant of the patent: **16.07.2025**
(21) Application number: 18166390.7
(22) Date of filing: 09.04.2018
(51) Int. Cl.: A61K 41/00, A61K 47/65, A61K 47/55, A61K 47/69, A61P 35/00

(54) **TUMOR-TARGETING PHOTOSENSITIZER-DRUG CONJUGATE, METHOD FOR PREPARING SAME AND PHARMACEUTICAL COMPOSITION FOR PREVENTING OR TREATING TUMOR CONTAINING SAME**
FOTOSENSIBILISIERENDES TUMOR-TARGETING-ARZNEIMITTEL-KONJUGAT, VERFAHREN ZUR HERSTELLUNG DAVON UND PHARMAZEUTISCHE ZUSAMMENSETZUNG ZUR VORBEUGUNG ODER BEHANDLUNG VON TUMOREN DAMIT
CONJUGUÉ PHOTOSENSIBILISATEUR-MÉDICAMENT CIBLANT LES TUMEURS, SON PROCÉDÉ DE PRÉPARATION ET COMPOSITION PHARMACEUTIQUE POUR LA PRÉVENTION OU LE TRAITEMENT DES TUMEURS LE CONTENANT

(30) Priority: 04.01.2018 KR 20180001168
(43) Date of publication of application: 10.07.2019
(73) Proprietor: Korea Institute of Science and Technology, Seoul 02792 (KR)
(72) Inventor: KIM, Kwangmeyung, 02792 Seoul (KR); KWON, Ick Chan, 02792 Seoul (KR); PARK, Juho, 02792 Seoul (KR)
(74) Representative: advotec.

(56) References cited:
- CN-A- 105 669 831
- US-A1- 2002 155 999
- JUAN CHEN ET AL: "Using the singlet oxygen scavenging property of carotenoid in photodynamic molecular beacons to minimize photodamage to non-targeted cells", PHOTOCHEMICAL AND PHOTOBIOLOGICAL SCIENCES, vol. 6, no. 12, 1 January 2007 (2007-01-01), GB, pages 1311, XP055540480, ISSN: 1474-905X, DOI: 10.1039/b706820d

## Description

### TECHNICAL FIELD

The present disclosure relates to a tumor-targeting photosensitizer-drug conjugate, more particularly to a photosensitizer-drug conjugate which can be selectively delivered to a tumor tissue, allows for selective activation of an anticancer agent by photostimulation and allows for specific treatment of a tumor cell by photodynamic therapy and the drug, a method for preparing the same and a pharmaceutical composition for preventing or treating a tumor containing the same.

### BACKGROUND

Cancer is the number one cause of death in most developed countries including Korea. It is one of the most important diseases that should be overcome. In addition to the three major therapeutic means surgery, chemotherapy and radiation therapy, immunotherapy, gene therapy and photodynamic therapy (PDT) are available. Photodynamic therapy is the next-generation therapy of selectively destroying cancer cells only using singlet oxygen and free radicals generated from a chemical reaction between a photosensitizer, light and oxygen, with no pain to the patients. However, this therapy is not accurately known even to health care providers due to limited literatures and clinical experiences. Although these methods can remove disease-causing cells by acting on the disease sites, they may exhibit cytotoxicity to normal sites, thereby causing the death of normal cells. In addition, they cannot perfectly cure cancers and cause severe pain to the patients.

To overcome this, various nanoparticles for photodynamic therapy have been developed and have drawn attentions as new anticancer agents for over a decade. However, many disadvantages such as difficulty in uniform preparation due to the complicated structure of the nanoparticles, complicated process and side effects due to high toxicity for normal cells make clinical application difficult.

Accordingly, development of a new anticancer agent for photodynamic therapy, which exhibits a superior tumor cell targeting ability, less side effects for normal tissues and high antitumor therapeutic effect, is necessary.

### [References of the Related Art]

### [Patent Document]

Korean Patent Registration No. 10-1756537.

Juan Chen et al. "Using the singlet oxygen scavenging property of carotenoid in photodynamic molecular beacons to minimize photodamage to non-targeted cells", Photochemical and Photobiological Siences, vol. 6, no. 12, 1 January 2007, page 1311, disclose a conjugate comprising the photosensitizer pyropheophorbide, a caspase-cleavable peptide and a carotenoid for use in the treatment of cancer by photodynamic therapy.

### SUMMARY

The present disclosure is directed to providing a very stable tumor-targeting photosensitizer-drug conjugate having specific activity for a tumor tissue and little cytotoxicity and a method for preparing the same.

The present disclosure is also directed to providing a composition for preventing or treating a tumor containing the photosensitizer-drug conjugate which is accumulated in a tumor tissue in the form of a stable prodrug nanoparticle structure under a physiological environment when intravenously administered and is activated by caspase-3 overexpressed in the tumor tissue when light is irradiated, thereby exhibiting a superior therapeutic effect of killing a tumor cell.

In an aspect, the present disclosure provides a tumor-targeting photosensitizer-drug conjugate wherein a photosensitizer, a peptide, a linker and an anticancer agent are conjugated sequentially, wherein the peptide is a peptide which is conjugated to one side of the photosensitizer and contains a sequence that can be specifically cleaved by caspase, the linker is conjugated to one end of the peptide and connects the peptide with the anticancer agent.

The linker is p-aminobenzyloxy carbamate (PABC).

The anticancer agent is monomethyl auristatin E.The photosensitizer is chlorin e6.

The photosensitizer-drug conjugate is represented by Structural Formula 1:

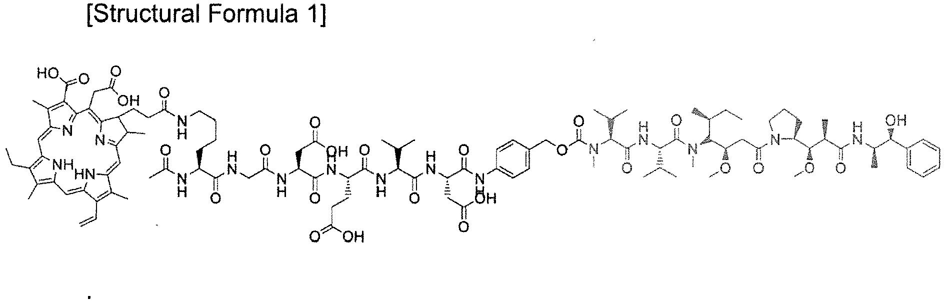

The tumor-targeting photosensitizer-drug conjugate may form a nanoparticle structure in a solution through self-assembly.

In another aspect, the present disclosure provides a method for preparing a photosensitizer-drug conjugate, including:
a) a step of, in a peptide containing a sequence that can be cleaved by caspase, substituting the hydrogen of amino acid residues excluding the site to which a linker is to be conjugated with an allyl group or an allyloxycarbonyl group;
b) a step of conjugating a linker to the C-terminal of the substituted peptide, wherein the linker is one or more selected from a group consisting of a small number of carbons, a peptide, polyethylene glycol (PEG) and p-aminobenzyloxy carbamate (PABC);
c) a step of preparing a drug conjugate by conjugating an anticancer agent to the linker;
d) a step of deprotecting the substituted peptide of the drug conjugate prepared in the step c) by substituting the allyl group or the allyloxycarbonyl group with hydrogen; and
e) a step of conjugating a photosensitizer to the N-terminal amino group of the deprotected peptide.

The peptide containing a sequence that can be cleaved by caspase in the step a) may be represented by SEQ ID NO 1.

In the step a) of preparing the substituted peptide, the carboxyl hydrogen of the side chain of the peptide containing SEQ ID NO 1 that can be cleaved by caspase may be substituted with the allyl group, the amino hydrogen of the side chain may be substituted with the allyloxycarbonyl group, and the N-terminal amine group may be protected with an acetyl group.

In another aspect, the present disclosure provides a pharmaceutical composition for use in preventing or treating a cancer, containing the photosensitizer-drug conjugate as an active ingredient.

The pharmaceutical composition containing the photosensitizer-drug conjugate may be selectively accumulated at a tumor site and induce selective death of a tumor cell when light is irradiated, the photosensitizer-drug conjugate may be cleaved by caspase-3 existing in the tumor cell and an anticancer effect containing exhibited as a drug may be released from the photosensitizer-drug conjugate which is in a prodrug form.

The cancer may be one or more selected from a group consisting of brain tumor, benign astrocytoma, malignant astrocytoma, pituitary adenoma, brain lymphoma, oligodendroglioma, craniopharyngioma, ependymoma, brain stem tumor, head and neck tumor, laryngeal cancer, oropharyngeal cancer, nasal cavity/paranasal sinus cancer, nasopharyngeal cancer, salivary gland cancer, hyopphayngeal cancer, thyroid cancer, oral cancer, breast tumor, small-cell lung cancer, non-small-cell lung cancer, thymus cancer, mediastinal tumor, esophageal cancer, breast cancer, male breast cancer, abdominal tumor, stomach cancer, liver cancer, gallbladder cancer, bile duct cancer, pancreatic cancer, small intestine cancer, large intestine cancer, anal cancer, bladder cancer, renal cancer, prostate cancer, testicular cancer, uterine cancer, cervical cancer, endometrial cancer, ovarian cancer, uterine sarcoma, squamous cell carcinoma and skin cancer.

The pharmaceutical composition may be injected by intravenous or topical administration.

The tumor-targeting photosensitizer-drug conjugate according to the present disclosure has little toxicity because it exists in the form of a prodrug nanoparticle structure in and ex vivo.

The photosensitizer-drug conjugate according to the present disclosure has a specific activity for a tumor tissue and is effectively accumulated in the tumor tissue. In addition, it exhibits the medicinal effect of the anticancer agent effectively as the DEVD peptide is cleaved by caspase-3 and the drug is released topically from the prodrug.

In addition, the photosensitizer-drug conjugate according to the present disclosure can be used for clinical applications without limitation because it exhibits tumor tissue-specific activity, stabilized cytotoxicity, etc. even though it does not contain an additional carrier.

Moreover, the photosensitizer-drug conjugate according to the present disclosure can achieve a superior anticancer effect even at low concentrations as compared to when the photosensitizer and the drug are used alone, because it can release the drug into the tumor cell through specific activity for caspase-3 and can exhibit a photodynamic therapeutic effect at the same time.

### BRIEF DESCRIPTION OF DRAWINGS

FIG. 1A is a reaction scheme whereby a photosensitizer-drug conjugate is synthesized in Example 1.
FIG. 1B shows a reversed-phase high-performance liquid chromatography measurement result of a photosensitizer-drug conjugate prepared in Example 1.
FIG. 1C shows an ESI-MS measurement result of a photosensitizer-drug conjugate prepared in Example 1.
FIGS. 2A-2C show ¹D proton NMR results of Ce6 (2A), MMAE (2B) and a photosensitizer-drug conjugate (CDM, 2C) and FIG. 2D shows an absorbance measurement result of Ce6, MMAE and a photosensitizer-drug conjugate (CDM).
FIG. 3A shows the structure of a photosensitizer-drug conjugate according to the present disclosure (CDM). Ce6 is colored blue, KGDEVD black, PABC green, and MMAE red.
FIG. 3B shows a process whereby a photosensitizer-drug conjugate according to the present disclosure is self-assembled to form a nanoparticle. The photosensitizer-drug conjugate of the present disclosure, which contains two hydrophobic drugs and a hydrophilic peptide linker, is self-assembled in a solution to form a nanoparticle.

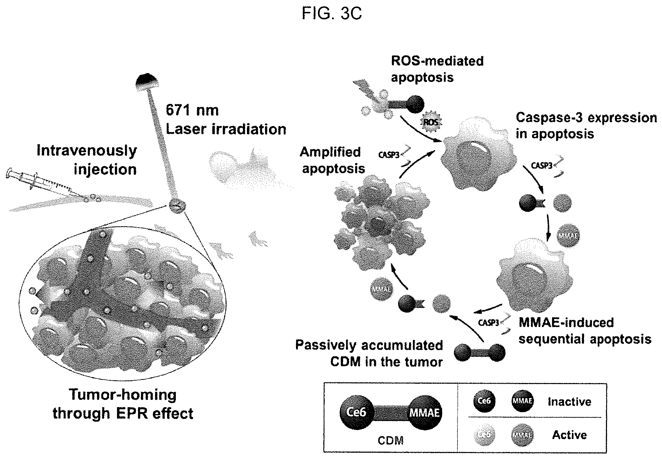

FIG. 3C schematically shows a principle of prevention or treatment whereby a photosensitizer-drug conjugate according to the present disclosure is activated in vivo and kills a tumor cell. The EPR effect and light-specific activation pathway of the photosensitizer-drug conjugate in a tumor cell are shown. The photosensitizer-drug conjugate is injected into the mouse tail vein and is selectively accumulated in the tumor tissue. The photosensitizer-drug conjugate is activated continuously and consistently by a laser (671 nm) from the initially activated site to the site where caspase-3 exists. That is to say, the cytotoxic effect of MMAE is exerted in the tumor cell which may or may not exist at the site where the laser is irradiated.
FIG. 4A shows a result of measuring the hydrodynamic diameter of a photosensitizer-drug conjugate of Example 1 (CDM) by dynamic light scattering (DLS).
FIG. 4B shows TEM images of MMAE, Ce6 and CDM, respectively.
FIG. 5 shows SEM (scanning electron microscopy) images showing that a photosensitizer-drug conjugate of Example 1 forms a specific nanoparticle with an average diameter of about 50-200 nm in physiological saline.
FIG. 6 shows the number of a photosensitizer-drug conjugate of Example 1 included in the volume of a nanoparticle formed by the photosensitizer-drug conjugate of Example 1 in a solution.
FIG. 7 shows a result of measuring the critical micelle concentration (CMC) of a CDM nanoparticle of Example 1 by the pyrene method.
FIG. 8 shows a result of measuring the fluorescence intensity ratio in the presence or absence of DMSO depending on the concentration of CDM or Ce6.
FIG. 9 shows a result of dissolving CDM in solutions having various salt concentrations and measuring the fluorescence intensity of Ce6 assembled therefrom in order to investigate the self-assembly of CDM depending on solution conditions.
FIG. 10 shows an HPLC result for CDM of Example 1 after incubating with caspase-3 for 15-120 minutes.
FIG. 11 shows an HPLC result obtained after preparing a mixture solution (CDM + caspase-3 + Inh) of a photosensitizer-drug conjugate of Example 1, caspase-3 and a caspase-3 inhibitor (Z-DEVD-FMK), a mixture solution (CDM + caspase-3) of the photosensitizer-drug conjugate of Example 1 and caspase-3 and a solution (CDM) containing the photosensitizer-drug conjugate of Example 1 only and performing incubation for 2 hours.
FIG. 12 shows confocal microscopy images obtained to investigate the intracellular distribution and cellular uptake of a photosensitizer-drug conjugate of Example 1 (CDM) in SSC7 cells after incubation for 6 hours.
FIG. 13 shows a result of measuring the cytotoxicity of CDM and MMAE used in combination with Ce6, CDM or caspase-3 in SCC7 cells. * represents statistical significance with respect to an untreated control group (p < 0.01).
FIG. 14 shows a result of measuring the generation of singlet oxygen from Ce6 and CDM in the presence of absence of DMSO.
FIG. 15 and FIG. 16 show a result of measuring the concentration of 1,3-diphenylisobenzofuran (DPBF) in Ce6 and CDM in the presence of 50% DMF depending on irradiation time and irradiation amount in order to investigate activity when a nanoparticle is not formed.
FIG. 17 shows a confocal immunofluorescence analysis result obtained using annexin V-FITC and PI (propidium iodide) by incubating SCC7 cells with CDM, before (0 h) and after (1 h, 3 h) laser irradiation.
FIG. 18 shows a western blot analysis result for SCC7 cells treated with Ce6 and a laser (Ce6 + laser), a laser only (laser), CDM only (CDM), MMAE only (MMAE) or CDM and a laser (CDM + laser) in order to detect immunoblots for activated caspase-3 and actin.
FIG. 19 shows a result of measuring intracellular caspase-3 activity. The activity of a cell extract of cleaving the colorimetric substrate Ac-DEVD-pNA was measured.
FIG. 20 shows a result of treating SCC7 cells with Ce6, CDM and MMAE and measuring cell viability before (laser (-)) and after (laser (+)) laser irradiation. * represents statistical significance (p < 0.01).
FIG. 21 shows images showing cytotoxic effect obtained by treating SCC7 cells with CDM and Ce6 and then irradiating a laser. The black circles indicate the sites irradiated with the laser.
FIG. 22 shows fluorescence images obtained by injecting CDM or Ce6 into a tumor animal model through the tail vein and imaging the whole body with time.
FIG. 23 shows a result of quantifying the amount of a fluorescent material accumulated in a tumor with time after injection of a drug into a tumor animal model.
FIG. 24 shows fluorescence images of tumors in the heart, kidney, spleen, lung and liver.
FIG. 25 shows a result of quantifying the fluorescence intensity of Ce6 and CDM from tumors and organs of a tumor animal model.
FIG. 26 shows a result of histological staining to compare the distribution and accumulation of Ce6 and CDM in a tumor tissue of a tumor animal model. DAPI is colored blue and Ce6 green.
FIG. 27 shows a result of measuring the plasma concentration of Ce6 and CDM with time after being injected into a tumor animal model (1 mg/kg).
FIG. 28 shows a fluorescence image obtained by preparing a tumor animal model (Balb/c nu/nu) by injecting SCC7 cells into the left and right flanks of a Balb/c nu/nu mouse, injecting CDM (0.5 mg/kg) into the tail vein of the tumor animal model when the tumor tissue reached to a certain level and irradiating a laser only to the right-side tumor tissue and a result of measuring fluorescence intensity.
FIG. 29 shows a result of injecting 0.5 mg/kg CDM or Ce6 into the tail vein of the tumor animal model (Balb/c nu/nu) of FIG. 28, irradiating a laser to the right-side tumor tissue only and measuring the size of both tumor tissues 15 days later.

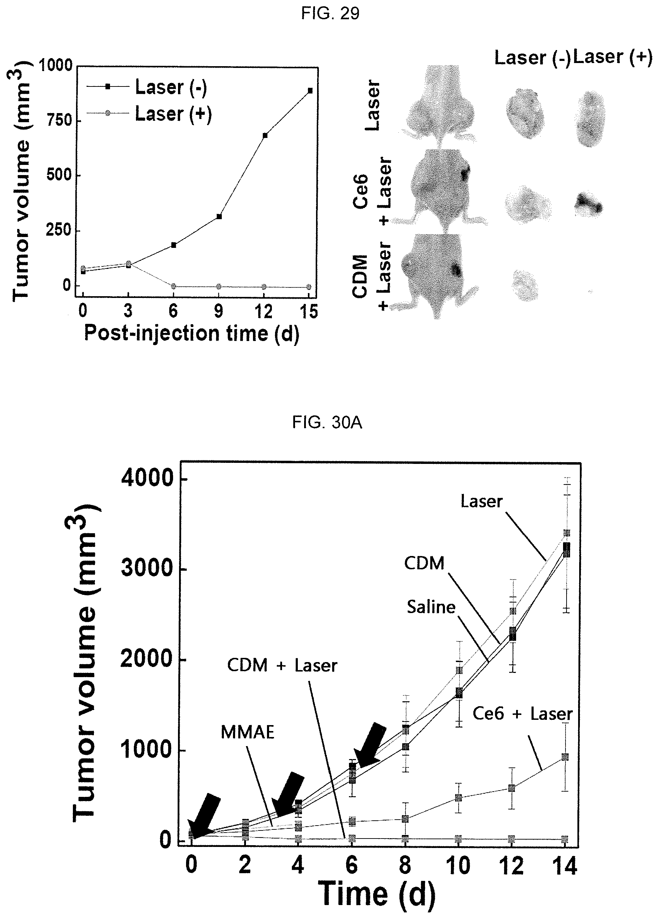

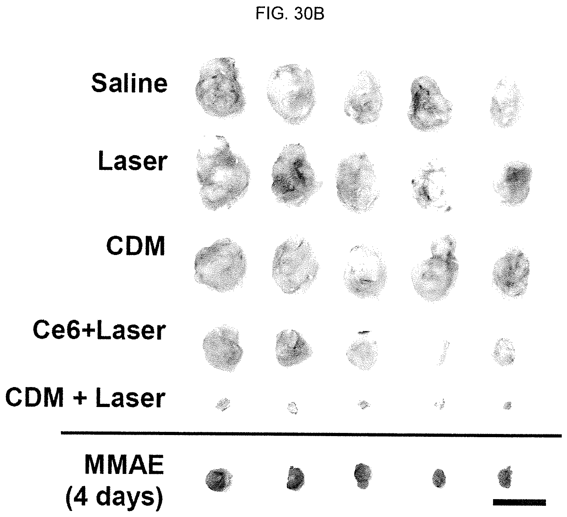

FIGS. 30A and 30B show a result of injecting a drug into a tumor animal model (C3H) and measuring the size of a tumor tissue with time. Groups were divided as follows: a saline group, a laser group treated only with a laser (10 min, 25 mW/cm²), a Ce6 + laser group treated with Ce6 (1 mg/kg) and a laser, a CDM group treated with CDM (0.25 mg/kg based on MMAE concentration) only, a MMAE group treated with MMAE (0.25 mg/kg) only, a CDM + laser group treated with CDM (0.25 mg/kg based on MMAE concentration) and a laser. A He-Ne laser (671 nm) was used and the laser was irradiated at 25 mW/cm² three times for 10 minutes after the injection of the drug (n = 6).
FIG. 31 shows a result of measuring the average weight of the tumor tissue extracted from the tumor animal model of FIGS. 30A and 30B.
FIG. 32 shows a H&E staining result of tumor slices extracted from the tumor animal model of FIGS. 30A and 30B. The scale bar represents 150 µm.
FIG. 33 shows a result of extracting a tissue from each group of the tumor animal model of FIG. 31 and conducting biopsy.
FIG. 34 shows a result of measuring the survival rate (%) of each group of a tumor animal model with time.
FIG. 35 shows a result of measuring the change in body weight (%) of each group of a tumor animal model with time.
FIG. 36 shows a result of measuring the change in body weight with time for a tumor animal model to which MMAE (50, 200, 500 µg/kg) or CDM (50, 200, 500 µg/kg based on MMAE concentration) was administered.
FIG. 37 shows a result of measuring the spleen weight (mg) of each group of a tumor animal model.
FIG. 38 shows a result of extracting the spleen from each group of a tumor animal model and analyzing the change in lymphocytes (lymphoid tissue; white pulp). In histological analysis, the oval white pulp corresponds to the lymphoid tissue.
FIG. 39 shows a result of counting the number of total white blood cells (WBCs) in plasma for a CDM group (0.5 mg/kg based on MMAE concentration) and a MMAE group (0.5 mg/kg).
FIG. 40 shows a result of measuring the blood neutrophil ratio (%) for a CDM group (0.5 mg/kg based on MMAE concentration) and a MMAE group (0.5 mg/kg).
FIG. 41 shows a result of measuring the plasma level of liver enzymes including aspartate aminotransferase (AST) and alanine aminotransferase (ALT) for a CDM group (0.5 mg/kg based on MMAE concentration) and a MMAE group (0.5 mg/kg).

### DETAILED DESCRIPTION OF EMBODIMENTS

Hereinafter, the present disclosure is described in detail.

Photodynamic therapy (PDT) is the most effective method for treating various cancers. The development of effective photosensitizers has been conducted for over a decade. Unlike other therapies such as chemotherapy or surgery, photodynamic therapy (PDT) has various advantages such as minimal invasion, high tumor-targeting ability and low toxicity. Although more advanced photosensitizer-based nanoparticles were developed, clinical application is limited due to the characteristic complicated structure and toxicity of the nanoparticles.

Despite the high potential in anticancer therapy, PDT has several problems associated with low efficiency and toxicity. To overcome this, technologies of forming a nanocomposite by binding a photosensitizer to a carrier or forming a nanoparticle and delivering a photosensitizer to a tumor site have been developed. These technologies aim to improve the effect of a drug by concentrating the drug to the tumor site using a self-assembled photosensitizer-based polymer. However, the therapeutic agent still has problems in preparation process and reliability due to the complicated structure of the nanoparticle and is limited in application because it exhibits cytotoxicity against normal cells.

Because the currently available photosensitizers have been developed to prepare potent photoactive biomaterials capable of treating various tumors, they are limited in clinical application and fail to solve the problems caused by complex structure.

The photosensitizer produces singlet oxygen when light is irradiated. Because it exhibits effect within the light-irradiated region only, its range of action is very limited. Therefore, therapeutic effect is not exerted for a tumor cell existing in the area where light cannot reach, a tumor cell which is not detected or a tumor cell with a large size. In addition, there are problems that intense pulsed light may cause several side effects to the skin tissue, the photosensitizer does not exhibit sufficient cytotoxicity and therapeutic effect cannot be exerted for tumors of various sizes because light cannot pass through the tissue. For these reasons, the therapeutic application of PDT is not extended and it is used limitedly only for the treatment of tumors of the head, neck and mouth.

MMAE (monomethyl auristatin E) is a superior synthetic antitumor agent which blocks the polymerization of tubulin even at very low concentrations (10⁻⁷-10⁻¹⁰ M), thereby inhibiting cell division. Despite this antitumor effect, it cannot be used as a drug due to non-specific activity and strong toxicity. Although some antibody-MMAE conjugates exhibiting low toxicity and superior effect have been developed recently, they are not being commercialized because of side effects.

With the development of nanotechnology, there has been rapid progress in the field of photodynamic therapy (PDT). In particular, nanoparticles for photodynamic therapy have drawn attentions as superior anticancer therapeutic agents for over a decade. However, despite superior functionality, the nanoparticles are disadvantageous in that they are very limited in applications due to the complexity and toxicity as described above. In order to solve these problems, the present disclosure presents a very stable conjugate in a prodrug form with a new structure, without using a chemical substance or a carrier.

An aspect of the present disclosure relates to a tumor-targeting photosensitizer-drug conjugate wherein a photosensitizer, a peptide, a linker and an anticancer agent are conjugated sequentially, wherein the peptide is a peptide which is conjugated to one side of the photosensitizer and contains a sequence that can be specifically cleaved by caspase, the linker is conjugated to one end of the peptide and connects the peptide with the anticancer agent.

The photosensitizer-drug conjugate of the present disclosure is a tumor therapeutic agent wherein the drug is activated even with a small quantity of light and is effectively released, thereby continuously killing nearby cancer cells. In addition, because the photosensitizer is conjugated with the enzyme-specific peptide and the linker, the photosensitizer-drug conjugate exhibits cytotoxicity and activity at the tumor site only. Moreover, the hydrophobicity of MMAE allows for formation of a spherical nanoparticle through interaction with Ce6 even in the absence of a nanocarrier, thereby providing a prodrug form with a very stable structure in vivo. The nanoparticle formed as the photosensitizer-drug conjugate of the present disclosure is self-assembled can be an alternative solution that can replace the existing nanoparticles for treating cancers.

Specifically, the photosensitizer is conjugated to the peptide-drug conjugate which is self-assembled under a physiological condition to form a nanoparticle having specific activity for caspase. When the photosensitizer-drug conjugate forms the nanoparticle, it exhibits caspase-3-specific anticancer activity as well as anticancer activity resulting from the photosensitive characteristics of PDT.

The photosensitizer-drug conjugate according to the present disclosure can be easily absorbed by a tumor cell, is accumulated in the tumor cell only passively and has caspase-3-specific activity. Therefore, it does not exhibit cytotoxicity in normal cells in vivo. That is to say, it is very stable because it exhibits no cytotoxicity in vivo. In addition, it exhibits very superior anticancer effect even when treated at extremely low concentrations (1-50 nM) as compared to the existing anticancer agents or PDT agents of the same concentrations. Furthermore, it is advantageous in that it can be activated even with a small quantity of light.

Because PDT is effective only for specific cancers in most cases, it cannot exhibit anticancer effect for metastatic or undetected cancers. However, the photosensitizer-drug conjugate according to the present disclosure exhibits effect not only for specific cancers but also for a broad range of cancers despite the absence of a carrier.

Because the photosensitizer-drug conjugate form a nanoparticle through self-assembly in vivo, a process for preparing into a nanoparticle form can be omitted and most problems of the existing PDT agents (clinical application, side effects, toxicity, etc.) can be solved.

The photosensitizer is chlorin e6, so that the photosensitizer-drug conjugate according to the present disclosure form a nanoparticle in a solution through interaction with other substances.

The peptide is represented by SEQ ID NO1 which has the most superior specific activity for caspase-3.
[SEQ ID NO 1]
   KGDEVD
[SEQ ID NO 2]
   GDEVD
[SEQ ID NO 3]
   DEVDG
[SEQ ID NO 4]
   DEVD

The linker is p-aminobenzyloxy carbamate (PABC), which forms a nanoparticle effectively in a solution through self-immolation.

The anticancer agent is monomethyl auristatin E.

The photosensitizer-drug conjugate is represented by Structural Formula 1:

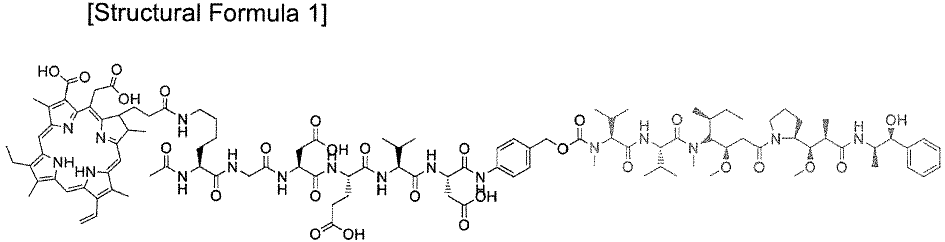

The tumor-targeting photosensitizer-drug conjugate forms a nanoparticle structure in a solution through self-assembly and is in a prodrug form which exhibits no cytotoxicity in vivo.

In the photosensitizer-drug conjugate with a new structure of the present disclosure, a peptide specific for caspase-3 (DEVD), a self-immolative linker, Ce6 capable of self-assembly and MMAE are conjugated. Its advantages are as follows. (i) It forms a spherical nanoparticle through self-assembly even without any nanocarrier and exists in a prodrug form exhibiting no toxicity to cells at normal times. (ii) It has light-induced targeting effect due to Ce6. (iii) It has specific activity against tumor cells. (iv) It has a self-immolative linker. (v) It exhibits very superior anticancer effect even at low concentrations.

DEVD is well known as a peptide that can be cleaved by caspase-3. The light-induced tumor targeting therapy of the present disclosure can increase apoptosis at the tumor site due to Ce6. Caspase-3 can selectively recognize the DEVD sequence existing in a substance and can enzymatically hydrolyze the bond between Ce6 and MMAE. To conclude, the prodrug form can be activated even with a very small quantity of light to exhibit anticancer effect and exists as a stable structure exhibiting no side effect of PDT and MMAE at normal times.

Another aspect of the present disclosure relates to a method for preparing a photosensitizer-drug conjugate including the following steps:
a) a step of, in a peptide containing a sequence that can be cleaved by caspase, substituting the hydrogen of amino acid residues excluding the site to which a linker is to be conjugated with an allyl group or an allyloxycarbonyl group;
b) a step of conjugating a linker to the C-terminal of the substituted peptide, wherein the linker is one or more selected from a group consisting of a small number of carbons, a peptide, polyethylene glycol (PEG) and p-aminobenzyloxy carbamate (PABC);
c) a step of preparing a drug conjugate by conjugating an anticancer agent to the linker;
d) a step of deprotecting the substituted peptide of the drug conjugate prepared in the step c) by substituting the allyl group or the allyloxycarbonyl group with hydrogen; and
e) a step of conjugating a photosensitizer to the N-terminal amino group of the deprotected peptide.

A specific process of the photosensitizer-drug conjugate of the present disclosure is illustrated in Scheme 1. A detailed description thereof is given below.

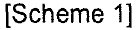

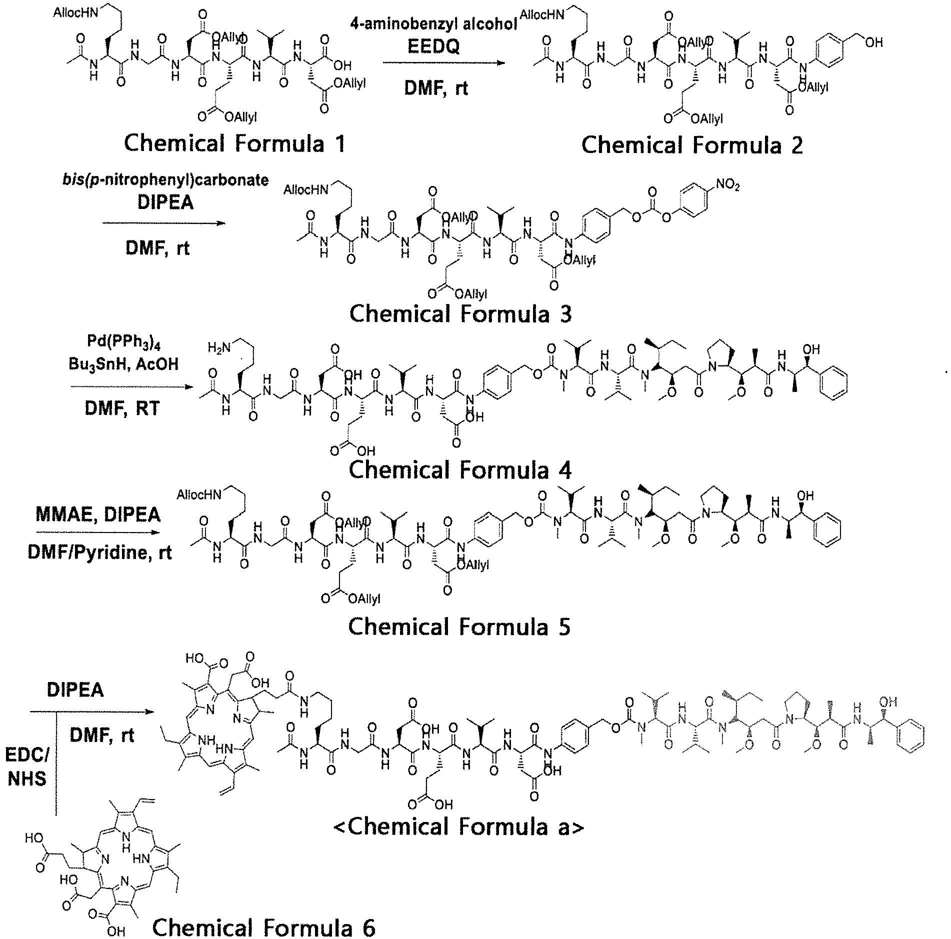

First, a) in a peptide containing a sequence that can be cleaved by caspase, the hydrogen of amino acid residues excluding the site to which a linker is to be conjugated is protected with an allyl group and an allyloxycarbonyl group. Specifically, the carboxyl hydrogen of the side chain of a peptide that can be cleaved by caspase represented by one of SEQ ID NOS 1-4 is substituted with an allyl group and the amino hydrogen of the side chain is substituted with an allyloxycarbonyl group to protect the N-terminal amine group with an acetyl group.

Then, b) in order to conjugate a linker to the C-terminal of the substituted peptide, the substituted peptide is treated with 4-aminobenzyl alcohol and EEDQ (2-ethoxy-1-ethoxycarbonyl-1,2-dihydroquinoline) at room temperature in the presence of DMF (dimethyl fumarate) and then treated with bis(p-nitrophenyl) carbonate and DIPEA, thereby synthesizing a peptide-linker conjugate of Chemical Formula 3.

Next, c) a drug conjugate is prepared by conjugating an anticancer agent to the linker. For this, the peptide-linker conjugate of Chemical Formula 3 is reacted with an anticancer agent and HOBt in the presence of anhydrous DMF. Then, after adding pyridine and DIPEA, the mixture is reacted at room temperature for 10-100 hours to synthesize a peptide-linker-anticancer agent conjugate represented by Chemical Formula 5.

Rather than directly conjugating Ce6 thereto, d) the drug conjugate prepared in the step c) is deprotected by substituting the allyl group or the allyloxycarbonyl group of the substituted peptide again with hydrogen.

Finally, e) the photosensitizer-drug conjugate according to the present disclosure is prepared by conjugating a photosensitizer to the N-terminal amino group of the deprotected peptide. For this, a photosensitizer activated with NHS is conjugated to the N-terminal amino group of the deprotected peptide.

Another aspect of the present disclosure relates to a pharmaceutical composition for use in preventing or treating a cancer, containing the photosensitizer-drug conjugate as an active ingredient.

The inventors of the present disclosure have made efforts to develop a new substance that can effectively prevent or treat cancers by inhibiting the growth of tumor cells and killing them. As a result, the inventors of the present disclosure have found a photosensitizer-drug conjugate which exists as a very stable structure exhibiting no cytotoxicity at normal times, thus exhibiting no effect of killing normal cells or normal tissues, but, in response to an external stimulus, experiences structural change and is successfully absorbed and accumulated in a tumor cell, thereby capable of selectively killing and inhibiting the growth of the tumor cell.

That is to say, through a new structure and combination of a photosensitizer and a drug, the photosensitizer-drug conjugate of the present disclosure exists as a very stable form at normal times, but, when light is irradiated or a specific condition is satisfied, it exhibits tumor cell-specific cell-killing and cell growth-inhibiting effects through structural change. Through toxicity and pharmacokinetic tests, it was confirmed that the photosensitizer-drug conjugate according to the present disclosure exhibits remarkably superior anticancer effect and tumor cell-targeting effect as compared to when the photosensitizer and the drug are used alone.

Specifically, when the photosensitizer is used alone, it exhibits anticancer effect only for specific cancers and cannot exhibit anticancer effect for undetected cancer cells. In addition, it has very low therapeutic effect because it cannot exhibit anticancer effect for tumor cells at the sites where light cannot reach. Moreover, because it remains for a long period of time in all cells without being degraded in vivo, it may cause negative effects after administration. When the drug is used alone, it may cause side effects such as necrosis of normal tissues because it exhibits cell-killing and cell growth-inhibiting effects not only for tumor cells but also for normal cells.

However, the present disclosure solves the above-described problems and, at the same time, exhibits remarkably superior tumor-specific anticancer effect.

The photosensitizer is chlorin e6, so that the photosensitizer-drug conjugate according to the present disclosure form a nanoparticle in a solution through interaction with other substances.

The peptide is represented by SEQ ID NO 1, which has the most superior specific activity for caspase-3.
[SEQ ID NO 1]
   KGDEVD
[SEQ ID NO 2]
   GDEVD
[SEQ ID NO 3]
   DEVDG
[SEQ ID NO 4]
   DEVD

The linker is p-aminobenzyloxy carbamate (PABC), which forms a nanoparticle effectively in a solution through self-sacrifice.

The anticancer agent is monomethyl auristatin E. The photosensitizer-drug conjugate is represented by Structural Formula 1:

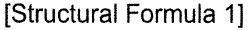

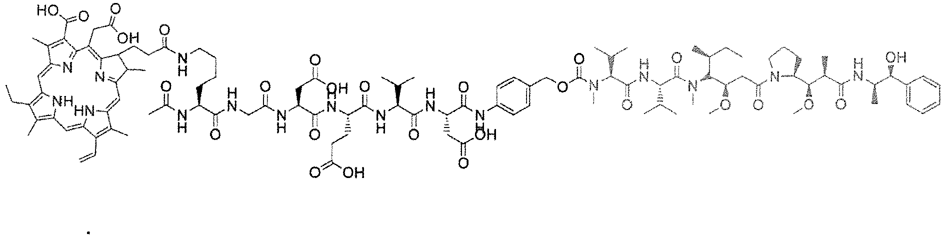

The tumor-targeting photosensitizer-drug conjugate forms a nanoparticle structure in a solution through self-assembly and is in a prodrug form which exhibits no cytotoxicity in vivo.

As described above, although various therapeutic agents have been developed for treatment of cancer, they are inapplicable to long-term treatment and there is a high risk of recurrence due to several problems. In addition, they cannot be used for patients because of side effects or staggering price.

Although photodynamic therapeutic agents have been developed to solve these problems, they show proven stability and therapeutic effect in vitro or in animal models only and there are many limitations in terms of light irradiation amount, tumor site, etc. in actual applications.

It was confirmed through test examples described below that the composition of the present disclosure is successfully absorbed and accumulated in SCC7 cells, thereby inhibiting the growth of the tumor cells and inducing the death of the cells.

In addition, as a result of investigating therapeutic effect in a tumor animal model, it was confirmed that the photosensitizer-drug conjugate according to the present disclosure can be used as a very effective anticancer agent.

In the present disclosure, the photosensitizer-drug conjugate is used as an active ingredient. Although the photosensitizer and the drug have been used respectively for treatment of cancer, actual clinical application was difficult due to several problems. However, because the photosensitizer-drug conjugate of the present disclosure exhibits anticancer effect specifically for tumor cells and acts via a very stable mechanism, it can not only treat cancers but also have broad therapeutic effect even for undetected cancers. Therefore, it exhibits remarkably superior anticancer effect, cellular absorption and uptake, specificity, etc. as compared to when the photosensitizer and the drug are used alone.

In addition, the composition can prevent or treat cancers by inhibiting the growth of cancer cells and inducing the death of the cancer cells.

The concentration of the active ingredient in the composition of the present disclosure needs not be limited particularly. The effect of improving, treating or preventing cancer by inhibiting the growth of cancer cells and inducing the death of the cancer cells may be achieved if the concentration is 1 nM or higher, specifically 10 nM or higher.

In the present disclosure, the expression 'containing (comprising) as an active ingredient' means that the photosensitizer-drug conjugate of the present disclosure is contained in an amount sufficient to achieve the effect or activity of treating or preventing cancer.

The pharmaceutical composition for use in preventing or treating a cancer containing the photosensitizer-drug conjugate as an active ingredient may contain the photosensitizer-drug conjugate in an amount of, for example, 0.001 mg/kg or more, specifically 0.1 mg/kg or more, more specifically 10 mg/kg or more, further more specifically 100 mg/kg or more, even more specifically 250 mg/kg or more, most specifically 0.1 g/kg or more. Because the photosensitizer-drug conjugate form a prodrug nanoparticle in a solution and exists as a very stable state exhibiting no toxicity to cells, it exhibits no side effect to the human body even when it is administered in an excess amount. Therefore, the upper limit of the photosensitizer-drug conjugate contained in the composition of the present disclosure may be determined adequately by those skilled in the art.

The pharmaceutical composition may be prepared by using, in addition to the active ingredient, a pharmaceutically suitable and physiologically acceptable adjuvant. As the adjuvant, an excipient, a disintegrant, a sweetener, a binder, a coating agent, a swelling agent, a lubricant, a glidant, a flavor, etc. may be used.

For administration of the pharmaceutical composition, one or more pharmaceutically acceptable carrier may be contained in addition to the active ingredient.

The pharmaceutical composition may be formulated into a granule, a powder, a tablet, a coated tablet, a capsule, a suppository, a liquid, a syrup, a suspension, an emulsion, a medicinal drop, an injectable solution, etc. For example, the tablet or capsule may be prepared by binding the active ingredient to a pharmaceutically acceptable non-toxic inert carrier such as ethanol, glycerol, water, etc. If desired or necessary, a suitable binder, lubricant, disintegrant, colorant or a mixture thereof may be further included. The suitable binder includes a natural sugar such as starch, gelatin, glucose or β-lactose, a natural or synthetic gum such as corn syrup, acacia, tragacanth or sodium oleate, sodium stearate, magnesium stearate, sodium benzoate, sodium acetate, sodium chloride, etc., although not being limited thereto. The disintegrant includes starch, methyl cellulose, agar, bentonite, xanthan gum, etc., although not being limited thereto.

As the pharmaceutically acceptable carrier used in a liquid formulation, one or more of saline, sterile water, Ringer's solution, buffered saline, albumin injection, dextrose solution, maltodextrin solution, glycerol and ethanol, which are sterile and physiologically acceptable, may be used. If necessary, commonly used other additives such as an antioxidant, a buffer, a bacteriostat, etc. may be added. In addition, a diluent, a dispersant, a surfactant, a binder and a lubricant may be further added to prepare an injectable formulation such as an aqueous solution, a suspension, an emulsion, etc., a pill, a capsule, a granule or a tablet.

In addition, the pharmaceutical composition may be formulated depending on particular diseases or ingredients according to the method described in Remington's Pharmaceutical Science, Mack Publishing Company, Easton PA.

The pharmaceutical composition may be administered orally or parenterally. The parenteral administration may be achieved through intravenous injection, subcutaneous injection, intramuscular injection, intraabdominal injection, transdermal administration, intratumor topical injection, etc. Specifically, the pharmaceutical composition may be administered orally.

An adequate administration dosage of the pharmaceutical composition may vary depending on such factors as formulation method, mode of administration, age, body weight and sex of a patient, pathological condition, diet, administration time, administration route, excretion rate and responsiveness and an ordinarily skilled physician can easily determine and prescribe an administration dosage effective for the desired treatment or prevention. In a specific exemplary embodiment, the administration dosage of the pharmaceutical composition is 0.001-10 g/kg per day.

The pharmaceutical composition may be prepared into a single-dose or multiple-dose formulation using a pharmaceutically acceptable carrier and/or excipient according to a method that can be easily employed by those of ordinary skill in the art to which the present disclosure belongs. The formulation may be a solution in an oily or aqueous medium, a suspension, an emulsion, an extract, a powder, a granule, a tablet or a capsule and may further contain a dispersant or a stabilizer.

Hereinafter, the present disclosure will be described in more detail through examples. However, the following examples are for illustrative purposes only and it will be obvious to those of ordinary skill in the art that the scope the present disclosure is not limited by them.

### Materials and methods

### 1) Materials

Chlorin e6 (Ce6) was purchased from Frontier Scientific Inc. (Logan, USA). Ac-KGDEVD was purchased from Peptron (Daejeon, Korea). Bis(p-nitrophenyl) carbonate, 1-ethyl-3(3-dimethylaminopropyl)carbodiimide (EDC), 2-ethoxy-1-ethoxycarbonyl-1,2-dihydroquinoline (EEDQ), N,N-diisopropylethylamine (DIPEA), hydroxybenzotriazole (HOBt), N-hydroxysuccinimide (NHS), p-aminobenzyl alcohol and tetrakis(triphenylphosphine)palladium(0) were purchased from Sigma Chemical Co. (St. Louis, MO). Tributyltin hydride (Bu₃SnH) and glacial acetic acid were purchased from Acros (USA). Anhydrous dimethylformamide (DMF) and dimethyl sulfoxide (DMSO) were purchased from Merck (Darmstadt, Germany). Glucose-rich DMEM, fetal bovine serum (FBS) and penicillin-streptomycin were purchased from GIBCO (Grand Island, NY). All the chemicals were of analytical grades and used without further purification.

### 2) Characterization

The product prepared in Example 1 was analyzed by high-performance liquid chromatography (HPLC; TFA 0.1%, UV 214 nm, distilled water and acetonitrile). The analyte was purified by semi-preparative HPLC (Shimadzu, Kyoto, Japan) using an ODS-A reversed-phase column (YMC, Dinslaken, Germany) under a concentration gradient condition (water and CH₃CN containing 0.05% trifluoroacetic acid).

Ce6-KGDEVD-PABC-MMAE represented by Chemical Formula a prepared in Example 1 was analyzed by proton-NMR and MALDI-TOF MS (matrix-assisted laser desorption/ionization mass spectrometry).

Molecular structure was investigated with ChemBioDraw Ultra 12.0 (Cambridge Soft Corporation), PyMOL 1.7.0.1 (DeLano Scientific) or Discovery Studio 4.0. The Ce6-KGDEVD-PABC-MMAE represented by Chemical Formula a prepared in Example 1 was analyzed after dissolving in DPBS (Dulbecco's phosphate-buffered saline; 0.1 mg/mL). Hydrodynamic size and distribution were measured by dynamic light scattering (SZ-100, Horiba, Ltd., Japan) using a 532 nm DPSS laser.

Morphology was observed by transmission electron microscopy (Talos F200X, FEI Company, USA). All the samples were treated with a 1% uranyl acetate solution to obtain negatively stained microscopic images.

### 3) Fluorescence quenching assay in vitro

In order to investigate the critical micelle concentration of CDM synthesized in Example 1, the fluorescence self-quenching effect was measured by the pyrene method. Briefly, a 0.01-10 µM CDM solution was dissolved in distilled water and incubated for 24 hours after adding 0.5 µM pyrene. Next, emission spectra were obtained at 372 nm and 383 nm using a spectrophotometer (F-7000, Hitachi High-Technologies Corporation, Japan) with an excitation wavelength of 340 nm. Then, fluorescence from the CDM of Example 1 depending on concentration was investigated using a real-time optical imaging system (IVIS Lumina K, PerkinElmer Inc., USA). The CDM solution used in Example 1 was an aqueous solution in which CDM was dissolved at a concentration of 0.1-10 µM and then 10% DMSO or 5% SDS was added. Fluorescence images were obtained at excitation (660 nm) and emission (710 nm) wavelengths.

### 4) Evaluation of ROS production in vitro

The quantum yield of reactive oxygen species from Ce6 and CDM was evaluated through p-nitroso-N,N'-dimethylaniline (RNO) photobleaching. More specifically, Ce6 and CDM solutions were prepared using an aqueous mixture solution of 250 µM RNO and 30 mM L-histidine and 1% DMSO. After irradiating a 671 nm He-Ne laser to the solutions, absorption spectra were measured at 405 nm using a UV-vis spectrometer.

### 5) Evaluation of cellular uptake and apoptosis in vitro

Experiments were conducted using SCC7 cells as follows. Specifically, the cells were cultured in a medium containing 10% fetal bovine serum (FBS) and 1% antibiotic using a 5% CO₂ incubator at 37 °C. In order to observe the cellular uptake and behavior of Ce6 and CDM in the SCC7 cells, 2x10⁵ SCC7 cells were seeded onto a glass-bottomed 35 pi cell culture dish and a single layer was formed by culturing for 24 hours. The cultured cells were washed with PBS and incubated after adding a serum-free medium containing 10 µM Ce6 or CDM. After the incubation was completed, the cells were washed and fixed with a 2% paraformaldehyde solution. Then, the cells were stained with 4,6-diamidino-2-phenylindole (DAPI, Invitrogen, USA).

The apoptosis of the fixed cells was measured using an annexin V-FITC apoptosis detection kit (Sigma-Aldrich Co., USA). Specifically, after adding Ce6 or CDM and incubating for 6 hours, the medium was replaced and light was irradiated to the cells at 2.4 J/cm² using an IR lamp. Then, the light-irradiated cells were treated with trypsin and stained for 30 minutes with FITC-annexin V and propidium iodide (PI) according to the manufacturer's instructions. The fluorescence images of the stained cells were obtained using a confocal laser scanning microscope (Leica TCS SP8; Leica Microsystems, Germany). To obtain the fluorescence images, a He-Ne laser (633 nm) and a UV diode (405 nm) were used to excite CDM and DAPI and an Ar laser (458, 514 nm) was used to excite FITC-Annexin V and propidium iodide (PI).

### 6) HPLC analysis

HPLC analysis was conducted using various enzymes in order to confirm that CDM has specific activity for caspase-3. Specifically, after dissolving 10 µM CDM in a pH 7.4 caspase detection buffer (50 mM HEPES, 0.9% NaCl, 0.1% CHAPS, 10 mM DTT, 1 mM EDTA, 10% glycerol) and adding 500 ng/mL caspase-3, the mixture was incubated at 37 °C for 15-120 minutes. Finally, the mixture solution was analyzed by RP-HPLC (Agilent 1200 series, Agilent Technology, USA) equipped with a UV detector.

### 7) Quantitative analysis of caspase-3 expression

Caspase-3 colorimetric assay and western blot were conducted to quantify enzyme expression. First, SCC7 cells were cultured on a 100 mm² cell culture dish until 70% confluence and further cultured for 6 hours after adding a drug (500 nM). After replacing the medium, light was irradiated with 20 mW/cm² for 5 minutes. After culturing further for 3 hours, the cells were recovered for analysis of caspase-3. For immunoblotting assay, the cells were treated with anti-cleaved caspase-3 antibody (1 : 650) and anti-PARP antibody (1 : 1000) purchased from Cell Signaling Technology (Danvers, MA) as primary antibodies and then treated with HRP-conjugated anti-rabbit IgG and antimouse IgG (1 : 1000; R&D Systems) as secondary antibodies. The blotted membrane was analyzed with a chemiluminescence imager (LAS-3000; Fuji Photo Film, Japan). Colorimetric assay of caspase-3 was conducted using a microplate reader (VERSAmax^{™}, Molecular Devices Corp., USA) and a caspase-3 assay kit (Abcam, Cambridge, MA) according to the manufacturer's instructions.

### 8) Analysis of cytotoxicity in vitro

The cytotoxicity of a drug was evaluated by a colorimetric assay using the cell counting kit-8 (CCK-8; Dojindo Molecular Technologies, Inc., USA). Specifically, 1x10⁴ cells were seeded onto each well of a 96-well plate and cultured for 24 hours. Then, the cells were incubated with a drug at various concentrations for 24 hours. After removing the culture medium, the plate was washed twice with PBS. The cells were incubated for 30 minutes in a medium containing a 10% CCK-8 solution and absorbance was measured at 450 nm using a microplate reader (VERSAmax^{™}; Molecular Devices Corp., USA).

A colorimetric assay was conducted using an IR lamp and CCK-8 in order to evaluate the cytotoxicity of CDM upon exposure to light. Specifically, 1x10⁴ cells were seeded onto each well of a 96-well plate and cultured for 24 hours. Then, the cells were incubated with a drug at 500 nM for 6 hours after washing twice with PBS. After removing the culture medium, the cells were irradiated with light at 2.4 J/cm². The cells were then incubated for 30 minutes in a medium containing a 10% CCK-8 solution and absorbance was measured at 450 nm using a microplate reader.

After the light irradiation was completed, SCC7 cells were cultured until 80% confluence on a 60 mm cell culture dish. Then, after adding Ce6 or CDM (500 nM), the cells were incubated for 6 hours. After the incubation was completed, the culture medium was replaced. A laser was irradiated at 20 mW/cm² for 5 minutes only onto an area marked with a small circle on the culture dish. Then, the cells were treated with trypan blue for 1 minute.

### 9) Analysis of tumor-suppressing effect in animal model (in vivo)

In order to conduct an in-vivo animal experiment, 5-week-oil BALB/c nude mouse mice were used as an allograft tumor animal model (BALB/c). The tumor animal model (BALB/c) was prepared by preparing a cell suspension containing 1x10⁶ SCC7 cells and 80 µL of a medium and injecting it into the thighbone at the flank of the BALB/c nude mouse where the effect of respiratory movement is little. Then, the mice were bred for about 8 days until the tumor grew to a size of about 80 mm³. All the animal experiment was conducted according to the guideline of the KIST Institutional Animal Care and Use Committee (IACUC) and relevant regulations and approved by the IACUC.

### 10) Analysis of biodistribution in vivo

Near-infrared (NIR) fluorescence imaging was conducted using a real-time optical imaging system in order to evaluate biodistribution in vivo. After administering 0.5 mg/kg CDM or Ce6 to the tumor animal model through the tail vein, fluorescence images were obtained for 48 hours using an excitation filter (660 nm) and an emission filter (710 nm). The tumor animal model treated with the drug was euthanized 48 hours later and fluorescence analysis was conducted after taking out organs.

### 11) Analysis of caspase-3 expression in vivo

The tumor animal model prepared by administering various drugs as described in 9) was subjected to photodynamic therapy and the expression level of caspase-3 in vivo was monitored. For this, NIR fluorescence images were obtained using a probe which is activated by specifically reacting with caspase-3 (Cas3p; Cy5-GDEVD-BHQ3).

After injecting 0.25 mg/kg CDM into the tail vein of the tumor animal model, a 671 nm laser was irradiated 6 hours later. After 3 hours, Cas3p was injected and its biodistribution was measured using an excitation filter (640 nm) and an emission filter (710 nm).

### 12) Pharmacokinetic analysis in vivo

For comparative analysis of pharmacokinetic characteristics in vivo, a tumor animal model was prepared in the same manner as in 9) using C57/BL6 mice instead of BALB/c nude mice.

CDM or Ce6 was injected into the tail vein of the tumor animal model (C57/BL6) at a concentration of 1 mg/kg. After the injection, 20 µL of blood was taken from the tail vein of the tumor animal model (C57/BL6) over 12 hours at predetermined time intervals. The blood was immediately diluted to 5-fold with a 0.5 mg/kg low-molecular-weight heparin solution (DMSO:DIW cosolvent). Then, after transferring to a 96- well plate, the fluorescence intensity of the drug in the blood was measured by near-infrared (NIR) fluorescence quantification using a real-time optical imaging system equipped with a 660 nm excitation filter and a 710 nm emission filter. A calibration curve was constructed to analyze the concentration of the drug from the detected fluorescence intensity. The calibration curve was constructed by taking blood from an untreated tumor animal model and adjusting the concentration of the drug in the blood to 10⁻⁸-10⁻⁴.

### 13) Analysis of toxicity in vivo

Blood toxicity indices such as neutrophil ratio, absolute neutrophil count (ANC), total whole blood cell (WBC) count, AST and ALT were measured for the mice treated with CDM or MMAE in order to evaluate toxicity in the animal model.

The body weight change of the tumor animal model was measured every day after treatment with the drug. 0.5 mg/kg CDM or MMAE was injected into the tail vein of the mice (7-week-old, male). 5 days later, blood samples (400 µL) were taken from the tail vein of the tumor animal model. All the blood samples were stored at 4 °C and analyzed within a day in SCL (Seoul Clinical Laboratories, Korea).

### 14) Analysis of antitumor effect in vivo

For evaluation of therapeutic effect in vivo, tumor volume was measured for a tumor animal model for 14 days. When the tumor tissue grew to a size of about 80 mm³, the tumor animal model was evaluated by dividing into 6 groups as follows.

A saline group treated with physiological saline, a laser group treated with a laser only, a Ce6 + laser group treated with a laser and Ce6 (1 mg/kg), an MMAE group treated with MMA E (0.25 mg/kg) only, a CDM group treated with CDM (0.25 mg/kg based on MMAE concentration) only and a CDM + laser group treated with CDM (0.25 mg/kg based on MMAE concentration) and a laser.

After injecting the drug into the tail vein, light was irradiated to the CDM + laser group and the Ce6 + laser group using a 671 nm He-Ne laser (25 mW/cm² for 10 minutes with 6-hour intervals). Then, the tumor size was measured every other day.

### 15) Histological analysis ex vivo

For histological analysis, tumor tissues and organ tissues were taken from the in-vivo animal model after conducting antitumor growth analysis. The extracted tissues were washed with PBS and fixed with a 4% paraformaldehyde solution. Then, the tissues were stained with H&E (hematoxylin and eosin) and the stained tissues were embedded in paraffin and placed on glass slides after cutting into 4 µm thick slices. After removing paraffin, the tissues were stained with H&E and observed under an optical microscope (BX 51; Olympus, USA). In order to observe the accumulation of CDM or Ce6 in tumor tissues, tumor tissues were taken out 24 hours after intravenous injection. The tissues were cut into 10 µm thick slices, freeze-dried and then observed under a confocal laser scanning microscope.

### 16) Statistical analysis

Significant difference between the groups was statistically analyzed by the one-way ANOVA test. P < 0.05 was considered statistically significant (indicated by asterisks).

### Example 1. Synthesis of Ce6-KGDEVD-MMAE conjugate (CDM)

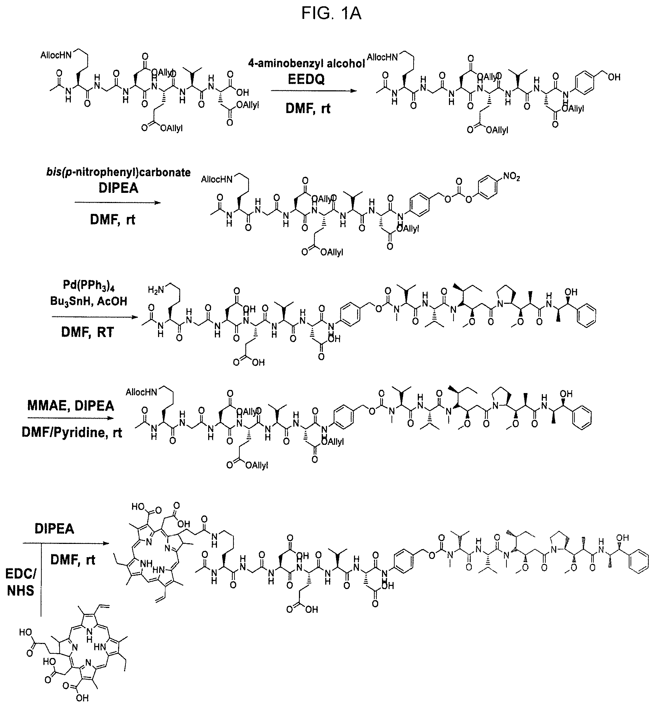

In order to demonstrate the hypothesis of the present disclosure, a photosensitizer-drug conjugate was prepared by synthesizing a caspase-3-specific MMAE prodrug containing Ce6 and a self-immolative linker. The synthesis was performed according to a series of processes described in Scheme 1 (see FIG. 1A).

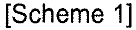

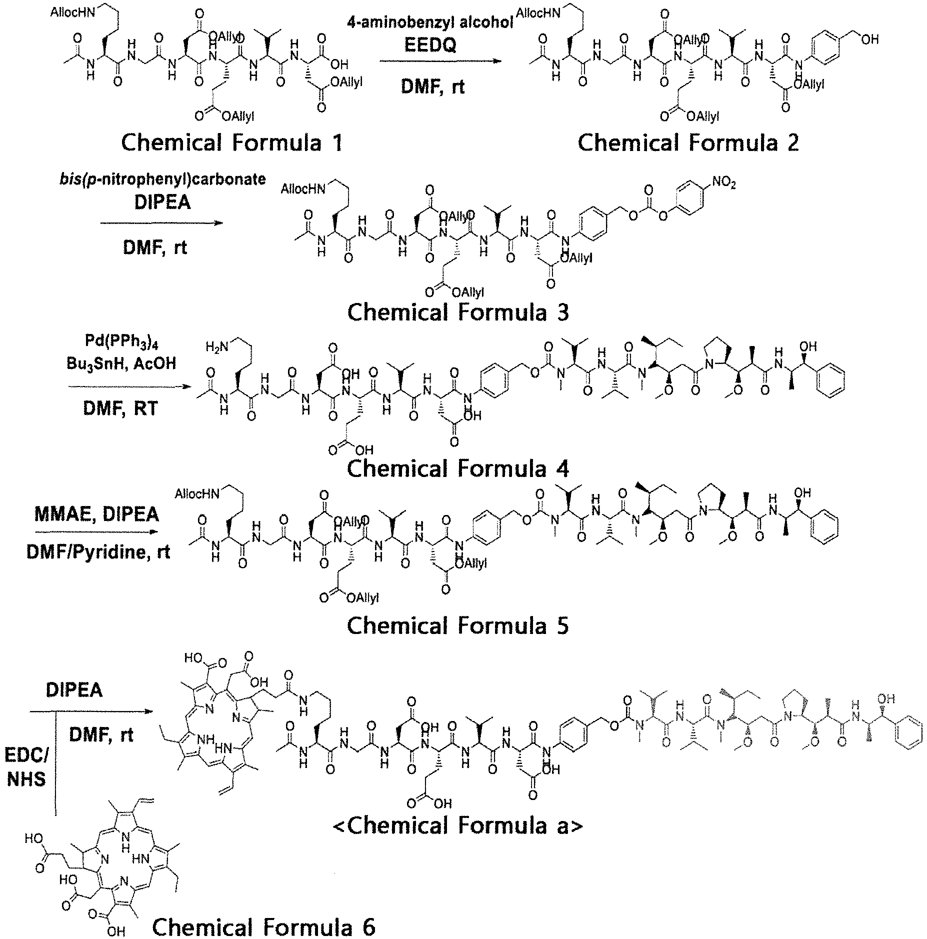

First, in order to conjugate a linker to (Ac)-KGDEVD, (Ac)KGDEVD (1 g, 1.10 mmol), p-aminobenzyl alcohol (0.67 g, 2.20 mmol, 2 eq) and EEDQ (0.27 g, 2.20 mmol, 2 eq) were mixed with anhydrous DMF (30 mL) and reacted overnight at room temperature. After pouring diethyl ether to the reaction solution, the formed precipitate was dried (yield: 99.6%). Then, the precipitate was dissolved and reacted at room temperature for 1 hour together with bis(p-nitrophenyl) carbonate (5 eq) dissolved in DMF (50 mL) and DIPEA (3 eq) dissolved in DMF (50 mL). Then, diethyl ether was poured again to synthesize a peptide-linker conjugate of Chemical Formula 3 as a precipitate (yield: 91.4%).

In order to conjugate MMAE to the peptide-linker conjugate of Chemical Formula 3, the peptide-linker conjugate of Chemical Formula 3 precipitate was dried to obtain a powder (653 mg) and then mixed with MMAE (478 mg, 1.2 eq) and HOBt (56 mg, 0.75 eq) in anhydrous DMF (40 mL). Then, a reaction mixture was obtained by adding pyridine (10 mL) and DIPEA (193 µL, 2 eq) and stirring at room temperature for 72 hours. Then, diethyl ether was poured to synthesize a peptide-linker-MMAE conjugate represented by Chemical Formula 5 as a precipitate.

In the peptide-linker-MMAE conjugate represented by Chemical Formula 5, the hydrogen of the amino acid residue of the peptide was protected with an allyl group or an allyloxycarbonyl group. To deprotect it, the peptide-linker-MMAE conjugate was dissolved in anhydrous DMF and stirred at 0 °C. After adding tetrakis(triphenylphosphine)palladium(0) (0.5 eq), tributyltin hydride (17.3 eq) and glacial acetic acid (20 eq) under nitrogen atmosphere and conducting reaction for 2 hours, the solution was filtered. The filtered solution was mixed with cold diethyl ether to precipitate the deprotected KGDEVD-PABC-MMAE. Then, NHS-activated Ce6 was added to DIPEA dissolved in anhydrous DMF solution together with the deprotected KGDEVD-PABC-MMAE and Ce6-(Ac)KGDEVD-PABC-MMAE of Chemical Formula a was synthesized by conducting reaction. The synthesized Ce6-(Ac)KGDEVD-PABC-MMAE was purified by C18 flash chromatography. A solution of 0.05% trifluoroacetic acid (TFA) and acetonitrile (ACN) (10-50% concentration gradient) was used as an eluent.

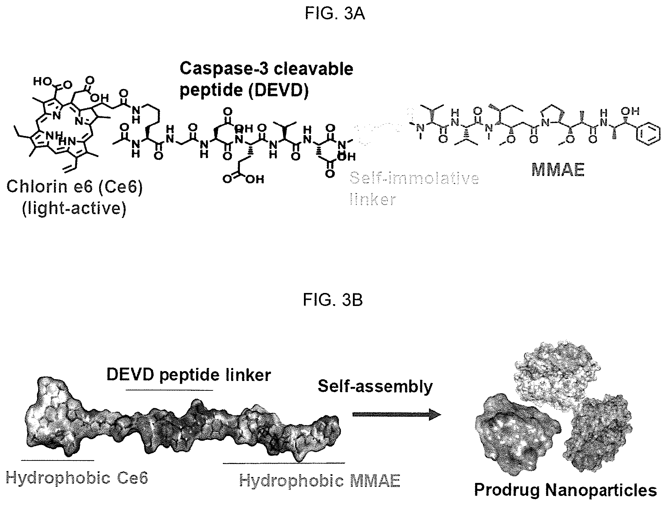

DEVD is specifically degraded by caspase-3 and is selectively degraded by apoptosis or in a tumor cell due to external factors such as light irradiation. MMAE and Ce6 were selected to resolve the complexity and limitation in doxorubicin quenching effect of the existing PDT-based therapy. The structure of the photosensitizer-drug conjugate is described in more detail in FIG. 3A.

The photosensitizer-drug conjugate of the present disclosure was designed to overcome the limitations of PDT. It is a prodrug-based self-assembling nanoparticle with a new structure (see FIG. 3B).

The photosensitizer-drug conjugate can be specifically and continuously activated even with a small quantity of light and exhibits an effective therapeutic or preventive effect because MMAE is released specifically only in tumor cells. The photosensitizer-drug conjugate of the present disclosure can solve most of the problems of the existing PDT because reactive oxygen species (ROS) and MMAE that induce apoptosis remarkably increase the therapeutic effect of PDT and the conjugate exhibits no toxicity at normal times but is activated in specific cells (FIG. 3C).

The photosensitizer-drug conjugate according to the present disclosure has an amphiphilic structure with two hydrophobic compounds on both ends and a hydrophilic peptide linker and form a nanoparticle in a solution through self-assembly. The molecular structure of the photosensitizer-drug conjugate consists of Ce6, a peptide (DEVD) that can be cleaved by caspase-3, a self-immolative linker and a MMAE. Before forming the conjugate, Ce6 has four modified pyrrole units on an aromatic ring having three carboxylic acids. But, after the conjugate is formed, it contains only two carboxylic acids. Therefore, the photosensitizer-drug conjugate can maintain its physical and chemical properties.

Meanwhile, because DEVE is a hydrophilic peptide which dissolves well in water, it plays an important role when the conjugate forms a nanoparticle in a solution through self-assembly.

The linker with an appropriate length avoids steric hindrance between caspase-3 and DEVD, thereby maintaining the characteristics of the prodrug. Although the potent anticancer agent MMAE did not receive attention in PDT, it was introduced as a new prodrug form in the present disclosure. Its structure is similar to those of general peptides but exhibits hydrophobicity, which is very favorable in forming a nanoparticle through self-assembly.

### Test Example 1. Characterization of photosensitizer-drug conjugate prepared in Example 1 (CDM)

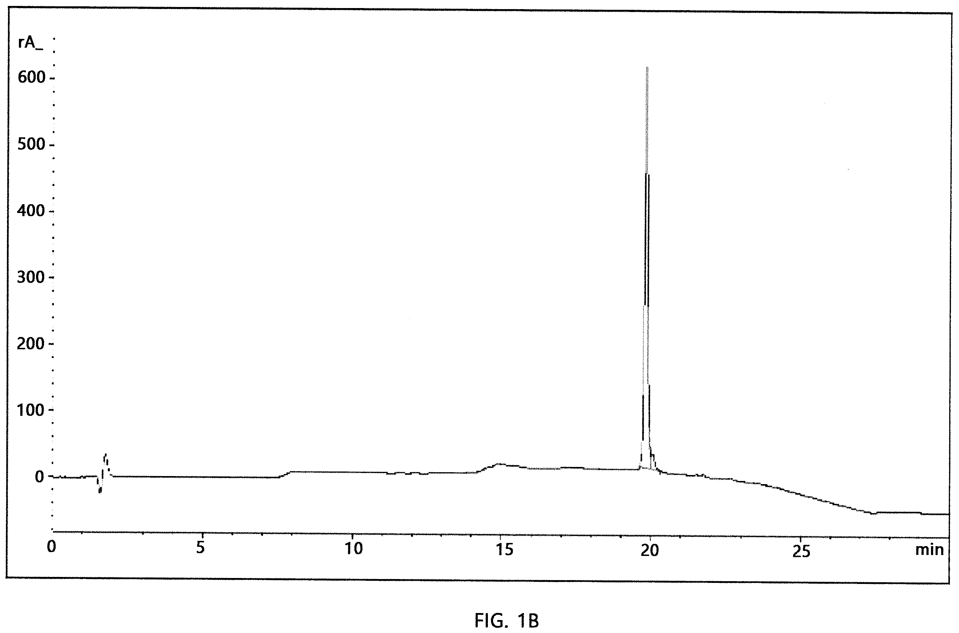

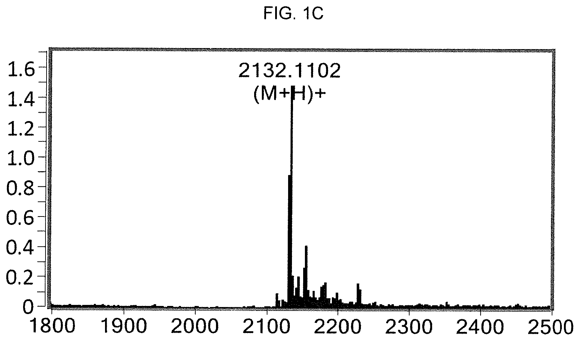

The final product was identified through in-vitro experiments using various methods. The product synthesized in each step was purified by reversed-phase high-performance liquid chromatography (RP-HPLC) and the purity is shown in FIG. 1B. The molecular weight of the photosensitizer-drug conjugate (CDM) was measured by ESI-MS (electrospray ionization mass spectrometry) (m/z calculated: 2131.1, found: 2131.1 Da) and the result is shown in FIG. 1C.

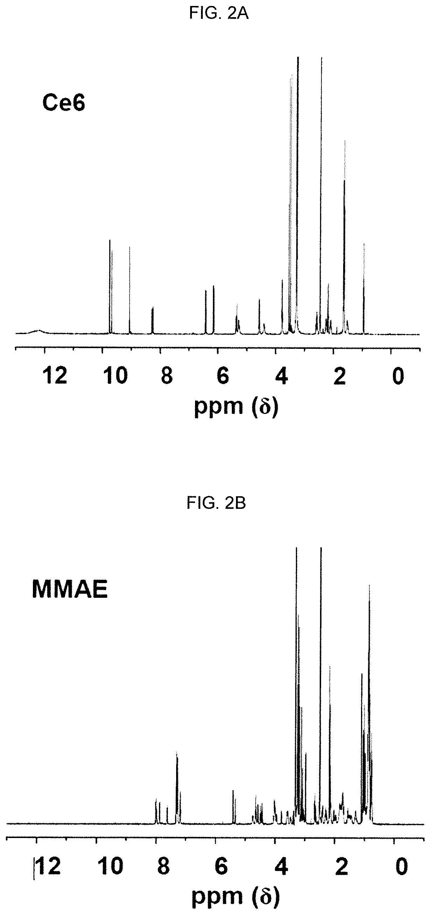

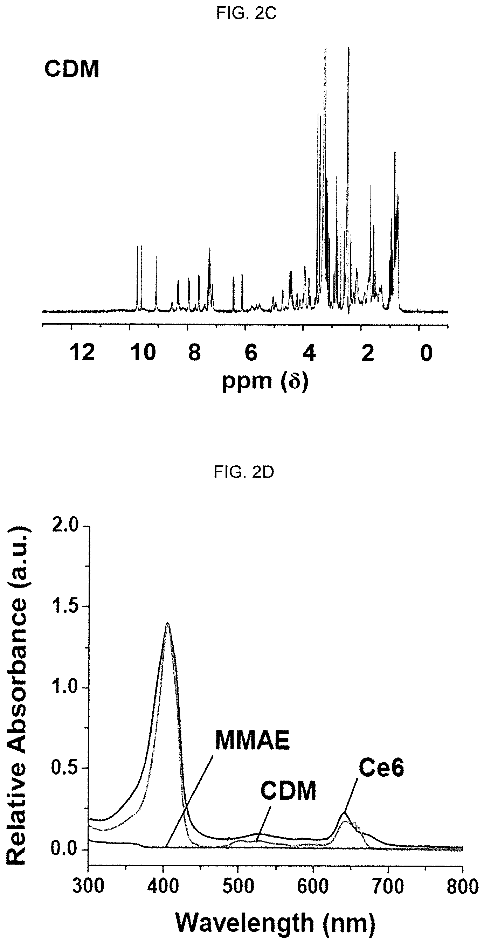

FIGS. 2A-2C show the ¹D proton NMR results of Ce6 (2A), MMAE (2B) and the photosensitizer-drug conjugate (CDM, 2C) and FIG. 2D shows the absorbance measurement result of Ce6, MMAE and the photosensitizer-drug conjugate (CDM). It was confirmed that, unlike Ce6 or MMAE, the photosensitizer-drug conjugate synthesized in Example 1 dissolves well in all of water, PBS and physiological saline.

### Test Example 2. Formation of self-assembled nanoparticle by photosensitizer-drug conjugate in solution

Because photosensitizer-drug conjugate (CDM) can form a nanoparticle through self-assembly, it does not require a carrier. Especially, the photosensitizer-drug conjugate (CDM) of the present disclosure is greatly advantageous in that it forms a nanoparticle stably while maintaining the characteristics of PDT and the prodrug. The peptide consists of aspartic acid (Asp) and glutamic acid (Glu) and has an appropriate moiety. Due to this, the photosensitizer-drug conjugate has amphiphilic property although it contains two insoluble drugs.

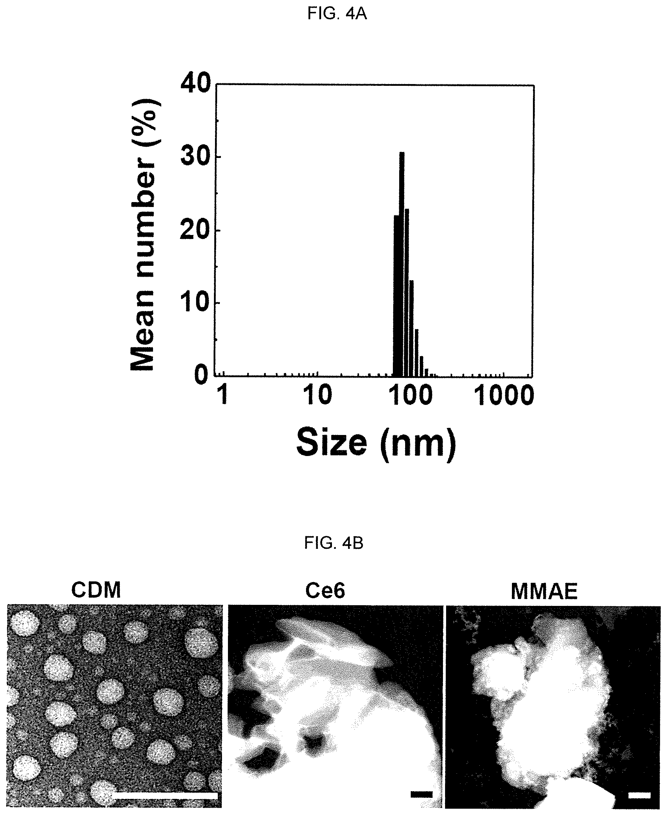

FIG. 4A shows a result of measuring the hydrodynamic diameter of the photosensitizer-drug conjugate of Example 1 (CDM) by dynamic light scattering (DLS) and FIG. 4B shows the TEM images of MMAE, Ce6 and CDM, respectively.

As seen from FIGS. 4A and 4B, the photosensitizer-drug conjugate successfully formed a nanoparticle through self-assembly and had an average diameter of 90.8±18.9 nm. The nano size of the photosensitizer-drug nanoparticle is advantageous in that it can be accumulated well in a tumor tissue through the EPR effect (FIG. 4A).

The morphology of the nanoparticle formed from the self-assembly of the photosensitizer-drug conjugate (CDM) in a solution was investigated by TEM (transmission electron microscopy). It was confirmed that nanoparticles were formed uniformly with a relatively circular shape in physiological saline when compared with Ce6 and MMAE (FIG. 4B).

From the analysis of the TEM images shown in FIG. 4B, it was confirmed that the self-assembled nanoparticle-based photosensitizer-drug conjugate had a uniform size distribution with an average diameter of about 52.6 ± 20.0 nm.

In contrast, when Ce6 or MMAE was dissolved in water alone, nanoparticles were formed only partly due to their water insolubility and poor physical properties. When Ce6 and MMAE were dissolved in water together, they spontaneously formed crystals in water through strong van der Waals interaction.

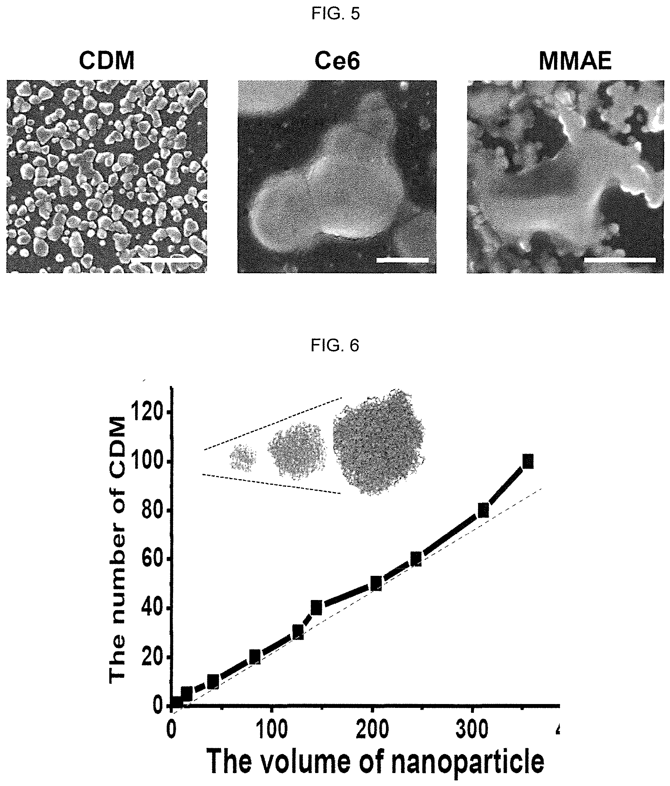

FIG. 5 shows SEM (scanning electron microscopy) images showing that the photosensitizer-drug conjugate of Example 1 forms a specific nanoparticle with an average diameter of about 50-200 nm in physiological saline and FIG. 6 shows the number of the photosensitizer-drug conjugate of Example 1 included in the volume of a nanoparticle formed by the photosensitizer-drug conjugate of Example 1 in a solution.

From FIG. 5 and FIG. 6, it was confirmed that the photosensitizer-drug conjugate formed a nanoparticle whereas Ce6 and MMAE did not form a nanoparticle when used alone.

For a nanoparticle-based pure prodrug, the theoretical encapsulation efficiency is 100% with respect to a nanoparticle prepared from a drug-drug conjugate. It is because it was assumed that no substance is contained except for sodium chloride (0.9% physiological saline). Therefore, the encapsulation efficiency is meaningless for a conjugate of a new structure other than the drug-drug conjugate.

It was confirmed through Discovery Studio and PyMOL dynamic simulation that 20,641 photosensitizer-drug conjugate (CDM) molecules are contained in one nanoparticle on average. That is to say, it was confirmed that about 20,000 photosensitizer-drug conjugate molecules were accumulated in the tumor tissue as prodrugs when one nanoparticle formed from the CDM according to the present disclosure through self-assembly reached the tumor tissue.

The critical micelle concentration (CMC) when the photosensitizer-drug conjugate according to the present disclosure was self-assembled to a nanoparticle in a solution was calculated.

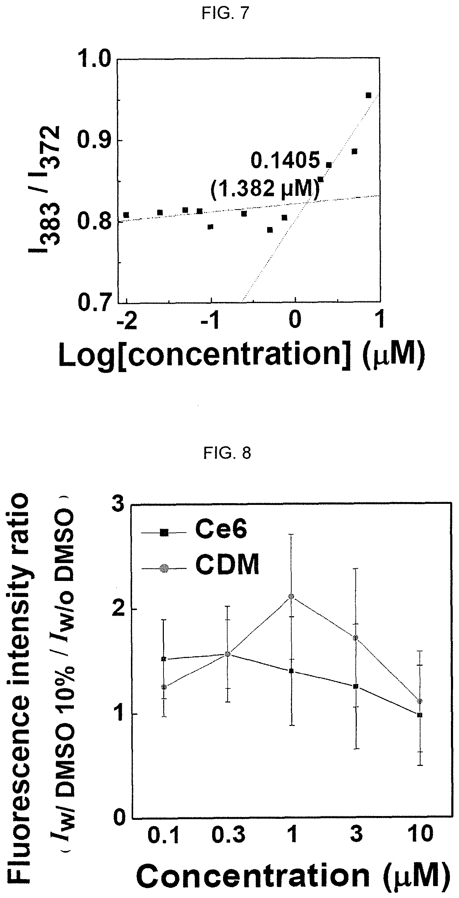

FIG. 7 shows a result of measuring the critical micelle concentration (CMC) of the CDM nanoparticle of Example 1 by the pyrene method. It can be seen that the photosensitizer-drug conjugate according to the present disclosure was self-assembled to a nanoparticle in a solution from a concentration of about 1.382 µM. This is much lower than the critical micelle concentration required for the existing CDM nanoparticle to form a self-assembled nanoparticle.

FIG. 8 shows a result of measuring the fluorescence intensity ratio in the presence or absence of DMSO depending on the concentration of CDM or Ce6. The fluorescence intensity ratio in the presence or absence of DMSO allows for evaluation of a nanoparticle indirectly. For a particle used as a PDT agent, the amount of emitted light varies depending on the densification of the particle. Considering the assembly of Ce6, the photosensitizer-drug conjugate of Example 1 was treated with 10% DMSO in order to induce structural change in a solution. As a result, CDM showed structural change depending on the solution whereas Ce6 showed no structural change. This experiment confirms again that the CDM exists as a densified nanoparticle in a solution.

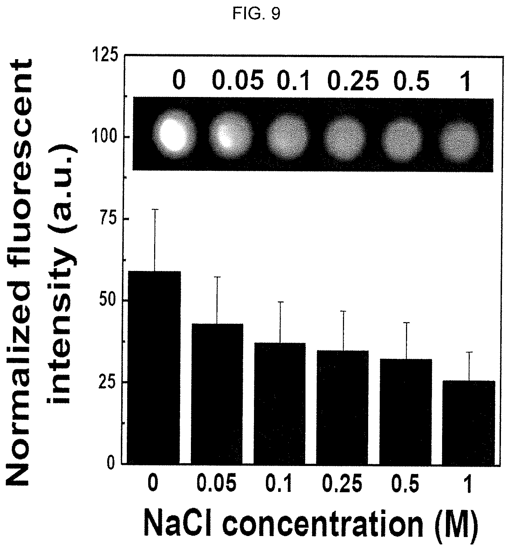

FIG. 9 shows a result of dissolving CDM in solutions having various salt concentrations and measuring the fluorescence intensity of Ce6 assembled therefrom in order to investigate the self-assembly of CDM depending on solution conditions. It can be seen that the photosensitizer-drug conjugate of Example 1 does not form a nanoparticle well under the sodium chloride environment due to strong ionic strength and the fluorescence intensity decreases. Therefore, it can be seen that the CDM according to the present disclosure forms a nanoparticle through self-assembly due to its hydrophobicity or amphiphilicity.

### Test Example 3. Evaluation of specificity of photosensitizer-drug conjugate of Example 1 (CDM) for caspase-3

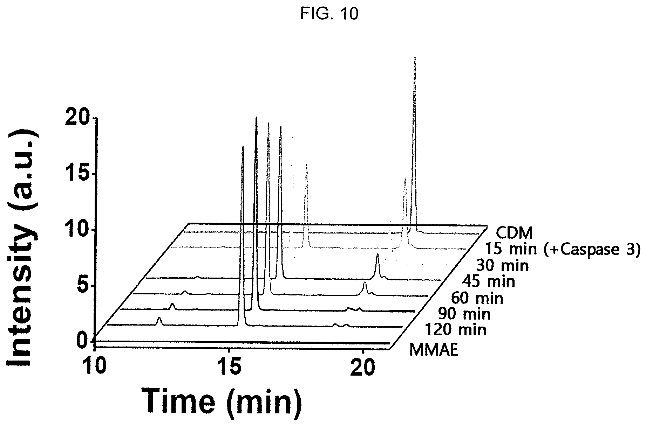

The photosensitizer-drug conjugate according to the present disclosure is advantageous in that it functions as a prodrug when it forms a nanoparticle through self-assembly. A caspase-3 buffer was prepared under a condition similar to the in-vivo apoptotic condition and treated with the photosensitizer-drug conjugate of the present disclosure for 120 minutes. The result is shown in FIG. 10.

FIG. 10 shows an HPLC result for the CDM of Example 1 after incubating with caspase-3 for 15-120 minutes. It can be seen that the photosensitizer-drug conjugate of Example 1 is effectively activated within 2 hours by caspase-3. The caspase-3-specific peptide sequence DEVD was successfully identified by the HPLC analysis.

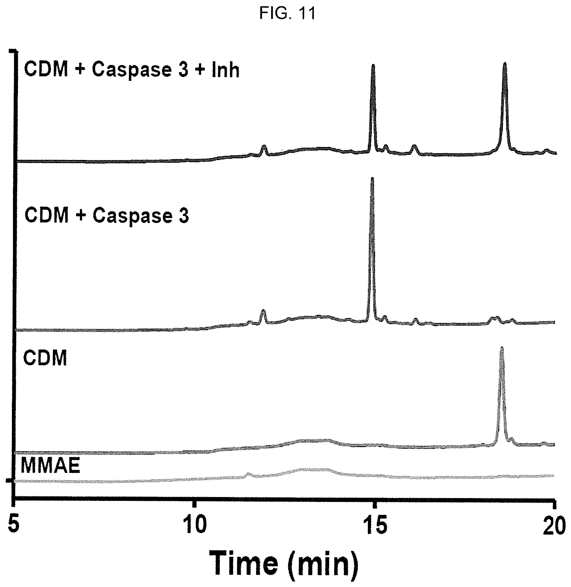

FIG. 11 shows an HPLC result obtained after preparing a mixture solution (CDM + caspase-3 + Inh) of the photosensitizer-drug conjugate of Example 1, caspase-3 and a caspase-3 inhibitor (Z-DEVD-FMK), a mixture solution (CDM + caspase-3) of the photosensitizer-drug conjugate of Example 1 and caspase-3 and a solution (CDM) containing the photosensitizer-drug conjugate of Example 1 only and performing incubation for 2 hours.

As seen from FIG. 11, the CDM was not completely activated by caspase-3 when it was treated with the caspase-3 inhibitor (Z-DEVD-FMK). Through this result, it was confirmed that the CDM according to the present disclosure is activated as it is specifically degraded in a tumor tissue by caspase-3.

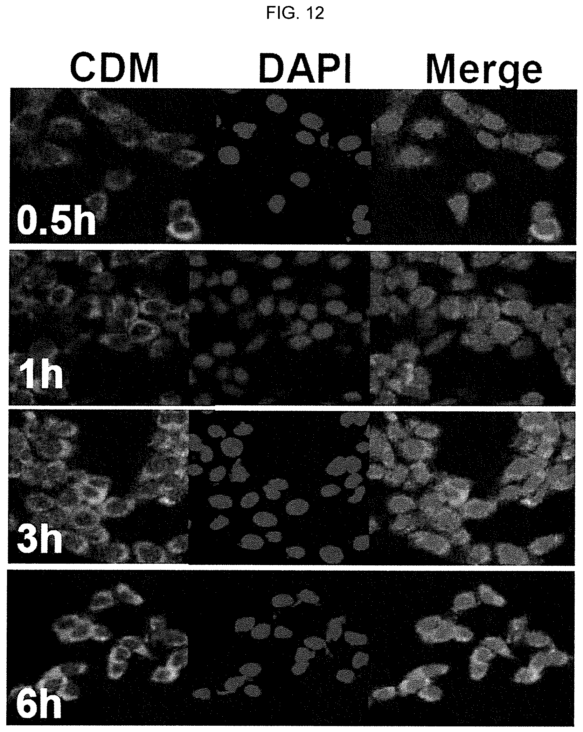

FIG. 12 shows confocal microscopy images obtained to investigate the intracellular distribution and cellular uptake of the photosensitizer-drug conjugate of Example 1 (CDM) in SSC7 cells after incubation for 6 hours. It was confirmed that, when SCC7 cells are treated with the photosensitizer-drug conjugate of Example 1 (CDM) for 0-24 hours at a concentration of 50 µg/mL, the CDM was accumulated in the cells in an enough amount within 3 hours.

The anticancer agent MMAE used in the present disclosure targets tubulin distributed in the cytoplasm. Although the active site of the CDM according to the present disclosure may be similar to that of Ce6, for the SCC7 cells treated with Ce6, because fluorescence was observed throughout the cell excluding the nucleus, it can be seen that it was not completely absorbed into the cell. Accordingly, a sufficient anticancer effect cannot be conveyed to the tumor cell when Ce6 is treated alone.

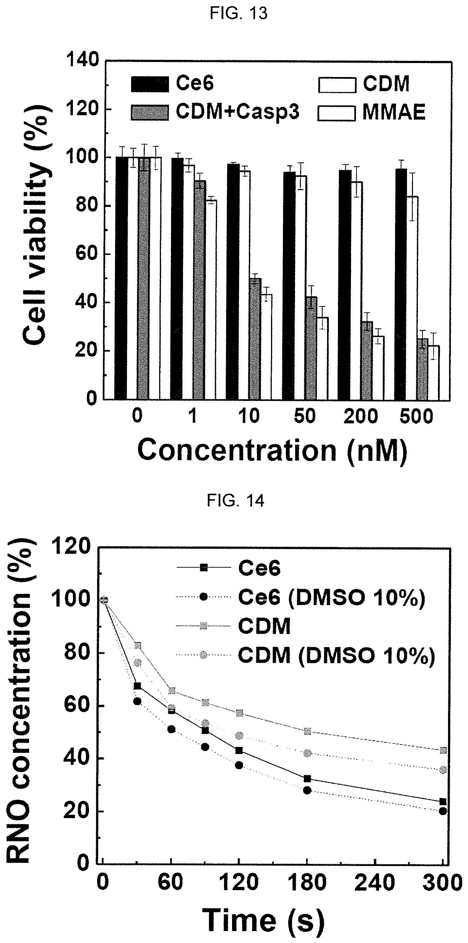

FIG. 13 shows a result of measuring the cytotoxicity of CDM and MMAE used in combination with Ce6, CDM or caspase-3 in SCC7 cells. * represents statistical significance with respect to an untreated control group (p < 0.01).

Referring to FIG. 13, it was confirmed that, in the cell viability assay using the CCK reagent, a combination of 10 nM CDM with caspase-3 effectively inhibited tumor cell growth to 50.1%. In contrast, treatment with 10 nM CDM not activated by caspase-3 showed little effect because the CDM existed as a prodrug form. Accordingly, it was confirmed that the CDM according to the present disclosure exhibits no toxicity to SCC7 tumor cells at normal times because it exists as a nontoxic prodrug form, but it is activated by light irradiation or caspase-3 and induces the death of the tumor cells.

When the cells were treated with Ce6 alone, the tumor cell inhibiting effect was exhibited only at relatively high concentrations and no effect was exhibited at 10 nM. When the cells were treated with the CDM according to the present disclosure at different low concentrations (50, 200, 600 nM) in the presence of caspase-3, the cell survival rate was decreased to 42.5, 32.5 and 25.5%, respectively. It can be seen that the CDM exhibits a comparable or better anticancer effect as compared to MMAE when treated at low concentrations.

### Test Example 4. Comparison of ROS producing effect of photosensitizer-drug conjugate of Example 1 (CDM) and Ce6

In PDT, Ce6 may lose its function due to chemical bonding or strong π-π interaction. Therefore, the ROS producing ability of the CDM according to the present disclosure was evaluated under different irradiation times and conditions.

First, the ROS producing ability of Ce6 and CDM was compared. The cytotoxicity reactive oxygen species produced by Ce6 and CDM was analyzed by measuring p-nitroso-N,N'-dimethylaniline (RNO) bleaching.

FIG. 14 shows a result of measuring the generation of singlet oxygen from Ce6 and CDM in the presence of absence of DMSO. It can be seen that, for CDM, the hydrophobic MMAE helps the CDM to form a nanoparticle in a solution through self-assembly but does not affect the ROS producing ability of Ce6. In addition, it was confirmed that more singlet oxygen is generated by CDM as compared to Ce6 of the same quantity. Specifically, the ROS producing effect of the CDM was maintained high as 83.1-58.2%.

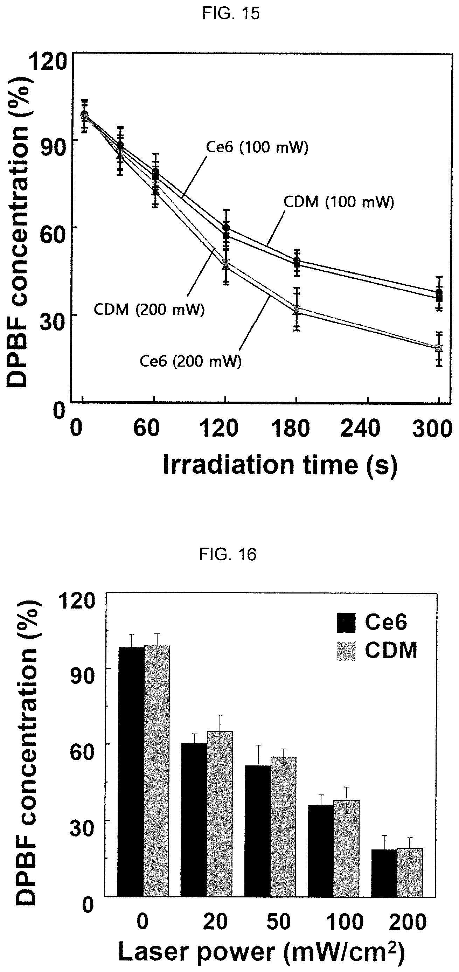

FIG. 15 and FIG. 16 show a result of measuring the concentration of 1,3-diphenylisobenzofuran (DPBF) in Ce6 and CDM in the presence of 50% DMF depending on irradiation time and irradiation amount in order to investigate activity when a nanoparticle is not formed. It can be seen that Ce6 and CDM show no difference depending on irradiation amount.

It was also confirmed that Ce6 and CDM show similar ROS producing ability depending on laser output (0-200 mW/cm²) under the same condition.

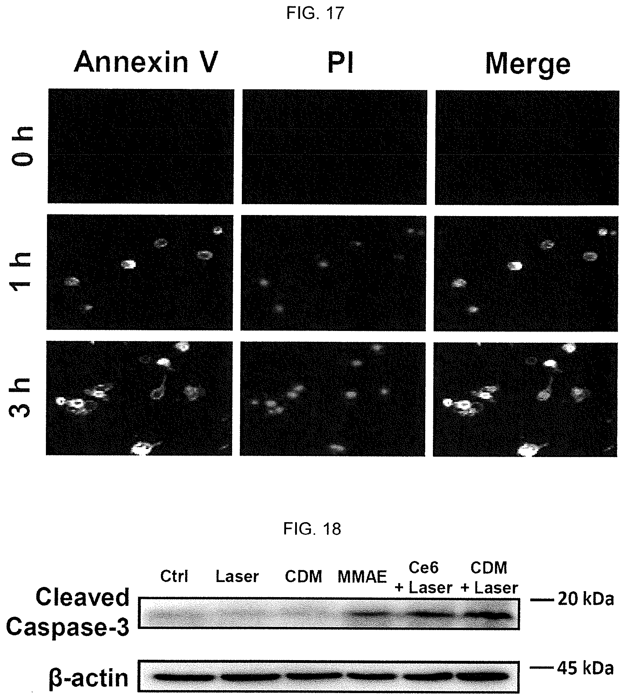

In order to investigate therapeutic effect for tumor cells, the cytotoxicity behavior of CDM upon light irradiation was observed by confocal laser scanning microscopy after staining cells with annexin V. FIG. 17 shows the confocal immunofluorescence analysis result obtained using annexin V-FITC and PI (propidium iodide) by incubating SCC7 cells with CDM, before (0 h) and after (1 h, 3 h) laser irradiation.

For the first 3 hours, the SCC7 cells treated with CDM did not show PI (propidium iodide) fluorescence signals, which means that apoptosis did not occur. As light was irradiated to the culture dish, the color of the cells began to change. This means that apoptosis began to occur as the CDM existing in the cells was stimulated by light. Through this, it was confirmed that the CDM according to the present disclosure is quickly and effectively absorbed and accumulated in the SCC7 cells and can induce apoptosis even with a small quantity of light.

It was investigated whether caspase-3 is upregulated when the cells treated with CDM are in apoptotic condition as light is irradiated. FIG. 18 shows a western blot analysis result for the SCC7 cells treated with Ce6 and a laser (Ce6 + laser), a laser only (laser), CDM only (CDM), MMAE only (MMAE) or CDM and a laser (CDM + laser) in order to detect immunoblots for activated caspase-3 and actin.

As seen from FIG. 18, the activated CDM decreased the level of pocaspase-3 and increased the intracellular concentration of caspase-3. This means that the expression level of caspase-3 is increased by apoptosis.

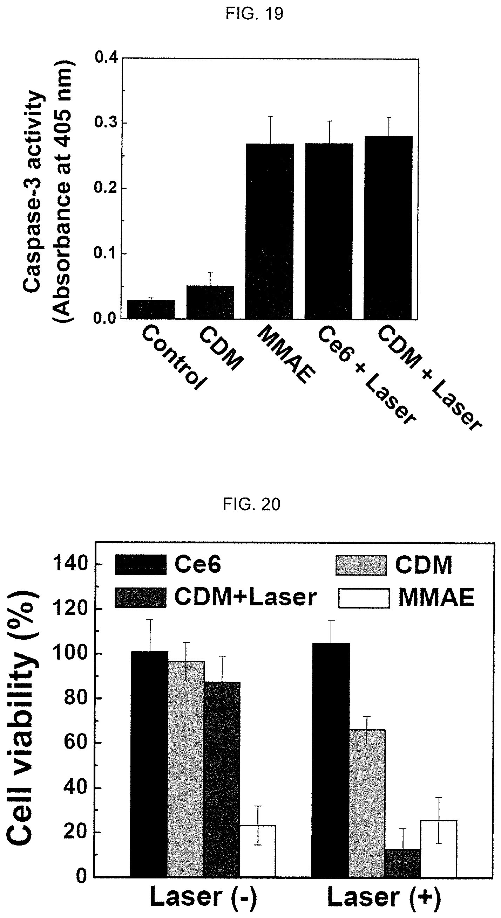

FIG. 19 shows a result of measuring the intracellular caspase-3 activity. The activity of the cell extract of cleaving the colorimetric substrate Ac-DEVD-pNA was measured.

Cell death caused by CDM and the level of caspase-3 released by laser irradiation were measured using a csapase-3 detection kit. As seen from FIG. 19, it was confirmed that MMAE increases the level of not only Ce6 and CDM but also caspase-3 in response to laser irradiation. This means that a continuous apoptosis process can be induced as the MMAE existing in the CDM is released. As a result, another CDM is further activated and, therefore, a consistent antitumor effect can be exhibited.

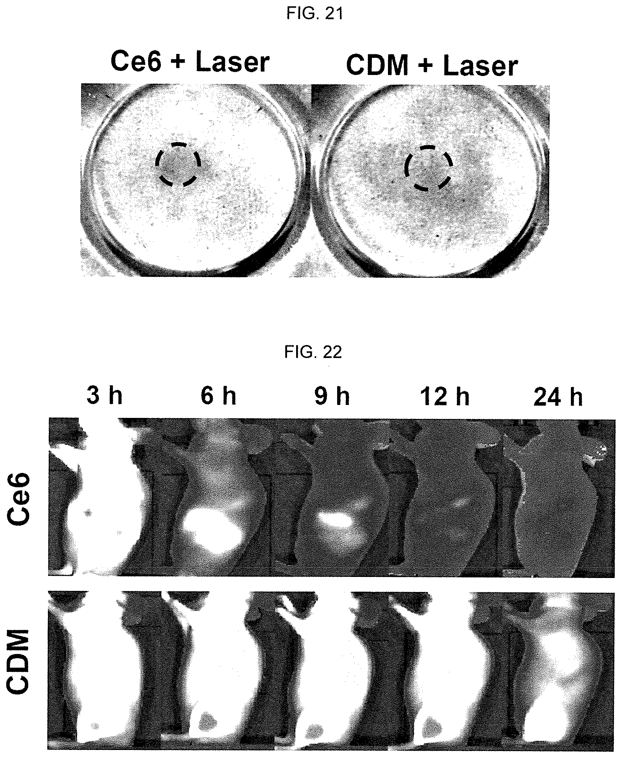

FIG. 20 shows a result of treating SCC7 cells with Ce6, CDM and MMAE and measuring cell viability before (laser (-)) and after (laser (+)) laser irradiation. * represents statistical significance (p < 0.01). FIG. 21 shows images showing cytotoxic effect obtained by treating SCC7 cells with CDM and Ce6 and then irradiating a laser. The black circles indicate the sites irradiated with the laser.

In FIG. 21, the stained cells treated with Ce6 and CDM are apoptotic cells that were killed after the laser irradiation. When the cells were treated with Ce6, only the cells treated with a laser were killed. In contrast, when the cells were treated with CDM, nearby cells were also killed effectively due to caspase-3.

In addition, when the tumor cells were killed by CDM as light was irradiated from outside (cell viability: 12.8 ± 9.2%), more cells were killed as compared to when they were treated with MMAE only (viability: 25.8 ± 10.1%) (FIG. 20).

It was confirmed that the CDM according to the present disclosure shows distinct structural change in response to external stimulation (light irradiation or caspase-3) and exhibits 2 time or higher cytotoxic effect even at much lower concentrations as compared to when the drugs are used alone. It may be because the DEVD peptide specifically recognized by caspase-3 is highly likely to be located on the surface of a nanoparticle, since the peptide is much more hydrophilic than Ce6 or MMAE.

### Test Example 5. Analysis of accumulation of self-assembled CDM in tumor model due to EPR effect

The CDM of the present disclosure prepared in Example 1 can be self-assembled to form a spherical nanoparticle, which also has the characteristics of a prodrug. In addition, when it exists in the form of a nanoparticle, it exhibits an increased in-vivo tumor-targeting effect through tumor-specific actions as compared to when it is simply dispersed.

The tumor-specific effect of the CDM according to the present disclosure in vivo was investigated through an in-vivo experiment. For the in-vivo experiment, a tumor animal model was prepared by injecting 1x10⁶ SCC7 cells into the left flank of a nude mouse. When the tumor size reached about 200 nm³, CDM and Ce6 were injected respectively through the tail vein of the tumor animal model (n = 3).

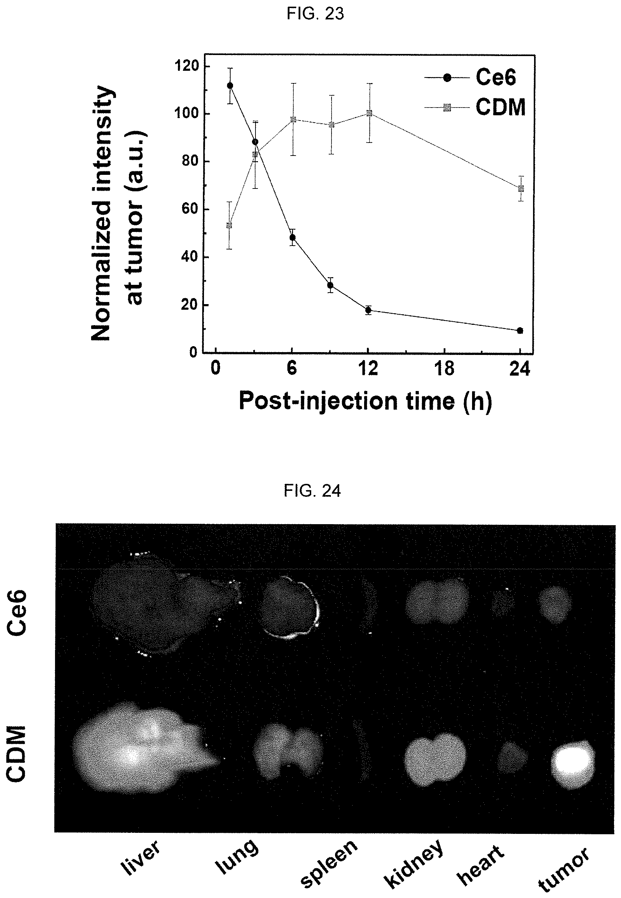

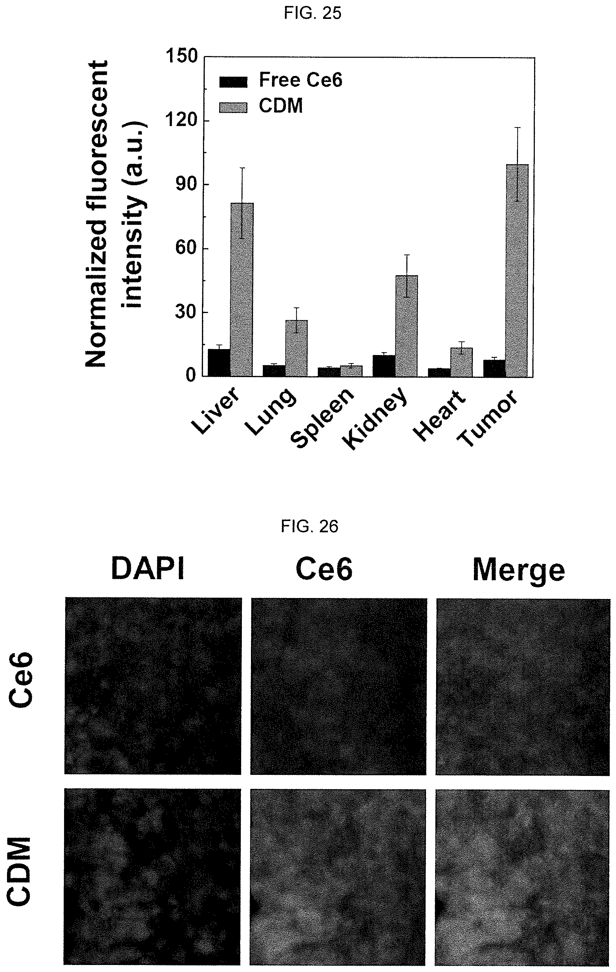

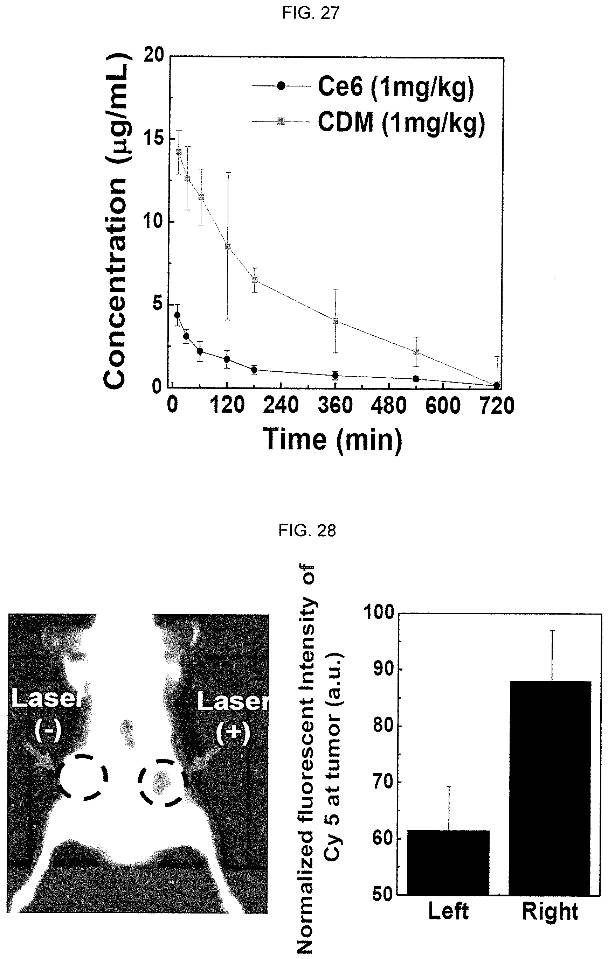

FIG. 22 shows fluorescence images obtained by injecting CDM or Ce6 into the tumor animal model through the tail vein and imaging the whole body with time. FIG. 23 shows a result of quantifying the amount of a fluorescent material accumulated in the tumor with time after injection of the drug into the tumor animal model. FIG. 24 shows fluorescence images of the tumors in the heart, kidney, spleen, lung and liver. FIG. 25 shows a result of quantifying the fluorescence intensity of Ce6 and CDM from the tumors and organs of the tumor animal model. FIG. 26 shows a result of histological staining to compare the distribution and accumulation of Ce6 and CDM in the tumor tissue of the tumor animal model. DAPI is colored blue and Ce6 green. FIG. 27 shows a result of measuring the plasma concentration of Ce6 and CDM with time after being injected into the tumor animal model (1 mg/kg).

It was expected from the foregoing experiments that, after being injected into the tumor animal model, CDM would form a nanoparticle in vivo through self-assembly and, thus, the tumor-targeting effect would be increased. As seen from FIG. 22, it was confirmed that CDM actually showed a remarkably superior tumor cell-targeting effect in vivo as compared to Ce6.

FIG. 23 shows a result of measuring the fluorescence intensity in the tumor tissue of the tumor animal model from FIG. 22. It can be seen that the CDM of the present disclosure (Example 1) exhibited the highest intensity for 6-12 hours in the tumor cell of the tumor animal model and the intensity decreased after 12 hours. In contrast, fluorescence was hardly detected in the tumor tissue when treated only with Ce6.

It was confirmed that the CDM of Example 1 was distinctly accumulated in the tumor tissue due to the EPR effect because its tumor-targeting ability was improved since it formed a nanoparticle through self-assembly. This means that the CDM according to the present disclosure forms a nanoparticle and functions when it is exposed to the physiological environment of the body.

As a result of injecting Ce6 and CDM (Example 1) respectively into the tumor animal model and measuring tumor tissues in other organs 24 hours later (FIG. 24), it was confirmed that the fluorescence intensity was high in the liver, lung, spleen, kidney, heart and tumor tissues of the CDM-injected tumor animal model. Through this, it can be seen that the tumor-specific accumulation of CDM is significantly increased as compared to the tumor-specific accumulation of Ce6.

The fluorescence intensity in the tumor tissue of the CDM-injected tumor animal model was 12.8-4.2 fold higher than that in the tumor tissue of the Ce6-injected tumor animal model (FIG. 25). Accordingly, it can be seen that the CDM (photosensitizer-drug conjugate) with a new structure according to the present disclosure has an improved EPR effect of being specifically activated, accumulated and targeted in the tumor tissue in vivo as compared to Ce6.

The CDM of the present disclosure is changed into a nanoparticle through self-assembly under the in-vivo condition. Therefore, it is expected that the pharmacokinetic (PK) characteristics of CDM will also change in vivo. To confirm this, the same amount (1 mg/kg) of CDM and Ce6 were injected into the blood of the tumor animal model and the blood levels of CDM and Ce6 were measured (FIGS. 26 and 27). At first, the blood level of CDM was higher than that of Ce6 but it was decreased down to the concentration of Ce6 with time. In association with FIG. 22, it can be seen that the blood level of CDM was decreased gradually for 6-12 hours while the amount of CDM accumulated in the tumor tissue was increased.

### Test Example 6. Antitumor effect of CDM in tumor animal model

After conducting anticancer therapy using CDM for a tumor animal model (Balb/c nu/nu or C3H), the effect of preventing or treating tumors was analyzed. SCC7 cells are commonly used to prepare a tumor animal model because they are potent tumor cells which grow fast and aggressively when injected into murines. In this example, a tumor animal model was prepared using the SCC7 cells and the potent effects of CDM could be measured accurately.

### 1) Tumor animal model (Balb/c nu/nu) experiment

First, in order to clarify the stimulation-specific therapeutic effect for a tumor animal model, the same tumor tissue was transplanted into different parts (left and right sides) of the same animal model and the two tumor tissues were compared.

A tumor animal model (Balb/c nu/nu) was prepared as described above in Materials and methods 9). 5 days after the intravenous injection of CDM, a single dose (30 mW//cm², 671 nm, 10 minutes) of laser was irradiated only to the right-side tumor tissue of the tumor animal model.

FIG. 28 shows a fluorescence image obtained by preparing the tumor animal model (Balb/c nu/nu) by injecting SCC7 cells into the left and right flanks of a Balb/c nu/nu mouse, injecting CDM (0.5 mg/kg) into the tail vein of the tumor animal model when the tumor tissue reached to a certain level and irradiating a laser only to the right-side tumor tissue and a result of measuring fluorescence intensity.

As seen from FIG. 28, when a probe (Cy5-GDEVD-BHQ3) activated by sensitively reacting with caspase-3 was injected to the tumor animal model which was treated with CDM and irradiated with a laser for the right-side tumor only, the fluorescence by caspase-3 was observed only in the right-side tumor. This clearly demonstrates that the laser irradiation induces apoptosis.

FIG. 29 shows a result of injecting 0.5 mg/kg CDM or Ce6 into the tail vein of the tumor animal model (Balb/c nu/nu), irradiating the laser to the right-side tumor tissue only and measuring the size of both tumor tissues 15 days later.

As a result of measuring the size of the two tumor tissues (FIG. 29), it was confirmed that there was distinct difference in the growth of the left-side and right-side tumors of the same tumor animal model. Through this, it can be seen that the effect of the CDM according to the present disclosure in vivo is changed by light-induced stimulation. In addition, it can be seen that CDM exhibits remarkably improved tumor cell-killing effect as compared to Ce6 because it is conjugated to MMAE.

### 2) Tumor animal model (C3H) experiment

FIGS. 30A and 30B show a result of injecting a drug into a tumor animal model (C3H) and measuring the size of a tumor tissue with time. Groups were divided as follows: a saline group, a laser group treated only with a laser (10 min, 25 mW/cm²), a Ce6 + laser group treated with Ce6 (1 mg/kg) and a laser, a CDM group treated with CDM (0.25 mg/kg based on MMAE concentration) only, a MMAE group treated with MMAE (0.25 mg/kg) only, a CDM + laser group treated with CDM (0.25 mg/kg based on MMAE concentration) and a laser. A He-Ne laser (671 nm) was used and the laser was irradiated at 25 mW/cm² three times for 10 minutes after the injection of the drug (n = 6).

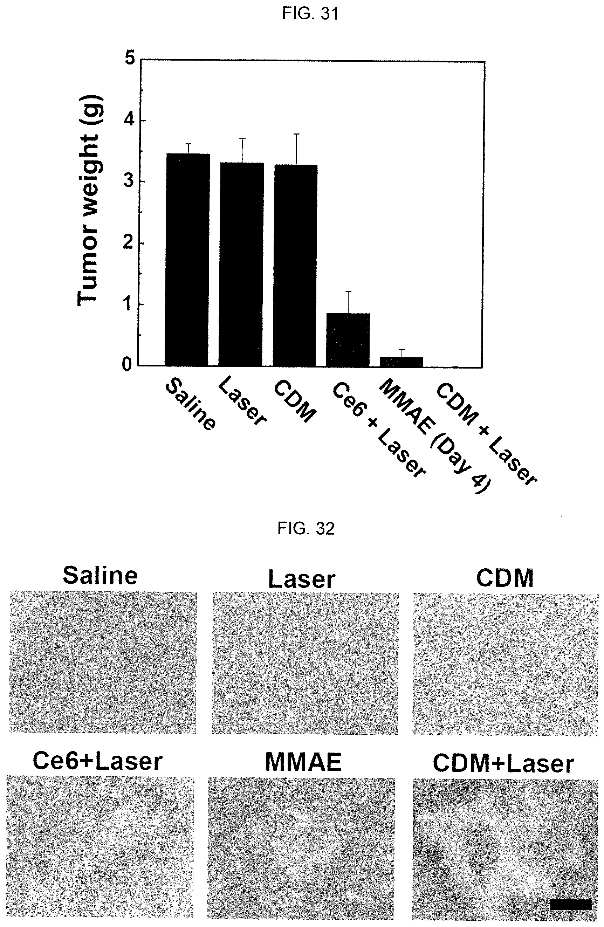

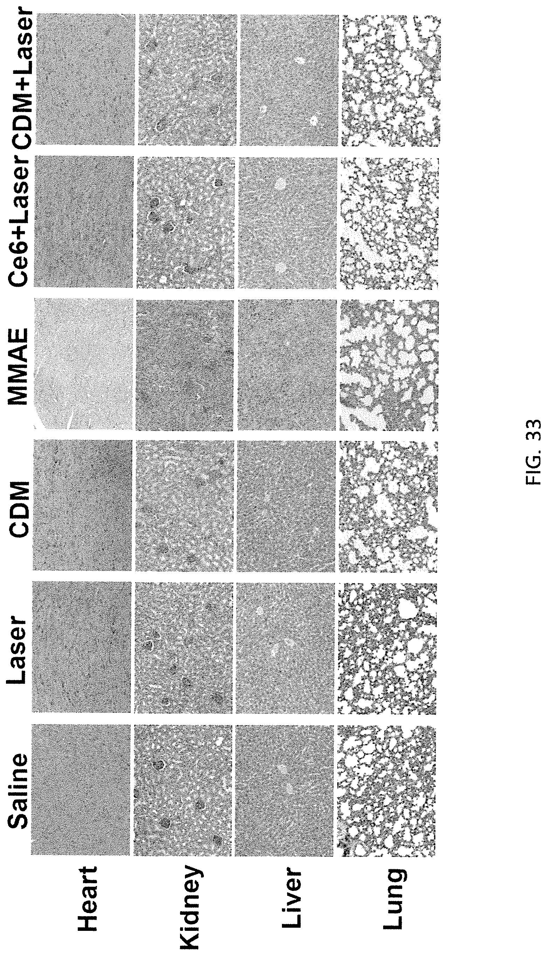

FIG. 31 shows a result of measuring the average weight of the tumor tissue extracted from the tumor animal model of FIGS. 30A and 30B. FIG. 32 shows a H&E staining result of tumor slices extracted from the tumor animal model of FIGS. 30A and 30B. The scale bar represents 150 µm. FIG. 33 shows a result of extracting a tissue from each group of the tumor animal model of FIG. 31 and conducting biopsy.

To describe in detail, each group was prepared by intravenously injecting Ce6 (1 mg/kg), MMAE (0.25 mg/kg) and CDM (0.5 mg/kg based on MMAE concentration) respectively into the flank of a C3H mouse bearing SCC7 tumor cells. As a result of analyzing the tumor size of each group with time (FIGS. 30A and 30B), the CDM group to which a laser was not irradiated showed no change in tumor size. The Ce6 + laser group treated with the photosensitizer Ce6 and the laser at the same time showed a tumor growth-inhibiting effect. It is thought that ROS generated by Ce6 causes tumor cell death by inducing oxidative stress. All the cells of the MMAE group died during the experiment because of too strong toxicity.

The CDM + laser group showed remarkably inhibited tumor growth as compared to other groups. The CDM + laser group treated with CDM and the laser at the same time showed no tumor growth and almost all tumor tissues were killed even with irradiation of a low-dose laser. Through this result, it was confirmed that the CDM according to the present disclosure exhibits very superior antitumor effect and therapeutic effect in the tumor animal model as compared to when the drugs are used alone, although it contains a very small amount of the photosensitizer.

In addition, the tumor weight of the CDM group, the laser group and the CDM + laser group of the tumor animal model (C3H) was measured. As a result, it was confirmed that the tumor weight of the CDM + laser group was remarkably decreased to 99.6% as compared to the CDM group and the laser group.

In order to investigate whether the tumor cells were actually killed, tissues were recovered from each group after the animal experiment was completed and biopsy was conducted by staining with H&E (FIG. 32). As a result, it was confirmed that the tumor cells were killed extensively for the CDM + laser group whereas the degree was very insignificant for the Ce6 + laser group.

In addition, organ tissues were extracted from each group of the drugtreated tumor animal model and biopsy was conducted (FIG. 33). As a result, no damage to other organs was observed for the Ce6 + laser group and the CDM + laser group.

To conclude these results, it was confirmed that the CDM according to the present disclosure specifically damages and kills tumor tissues only when treated together with a laser. Accordingly, it can be clearly seen that the CDM according to the present disclosure has a remarkably superior tumor-specific therapeutic and preventive effect as compared to other therapeutic agents.

### Test Example 7. Evaluation of cytotoxicity of CDM in animal model

Most of the recent Ce6-based nanoparticles or MMAE conjugates are limited to clinical application due to complexity and toxicity. Experiment was conducted to demonstrate that the CDM according to the present disclosure not only solves all the problems of the existing Ce6 nanoparticles and MMAE conjugates but also exhibits a more stable and better therapeutic effect.

First, a tumor animal model was prepared by transplanting SCC7 tumor cells into a C3H mouse. Details are described above in Materials and methods.

6 groups were prepared as follows by injecting drugs into the tail vein of the tumor animal model. 5 days after the drug injection, a single-dose (30 mW/cm², 671 nm, 10 minutes) laser was irradiated to the tumor tissue. The total treatment period was 14 days.

The groups were as follows: a saline group, a laser group treated with a laser (10 min, 25 mW/cm²) only, a Ce6 + laser group treated with Ce6 (1 mg/kg) and a laser, a CDM group treated with CDM (0.25 mg/kg based on MMAE concentration) only, a MMAE group treated with MMAE (0.25 mg/kg) only, a CDM + laser group treated with CDM (0.25 mg/kg based on MMAE concentration) and a laser. A He-Ne laser (671 nm) was used and the laser was irradiated at 25 mW/cm² three times for 10 minutes after the injection of the drug (n = 6).

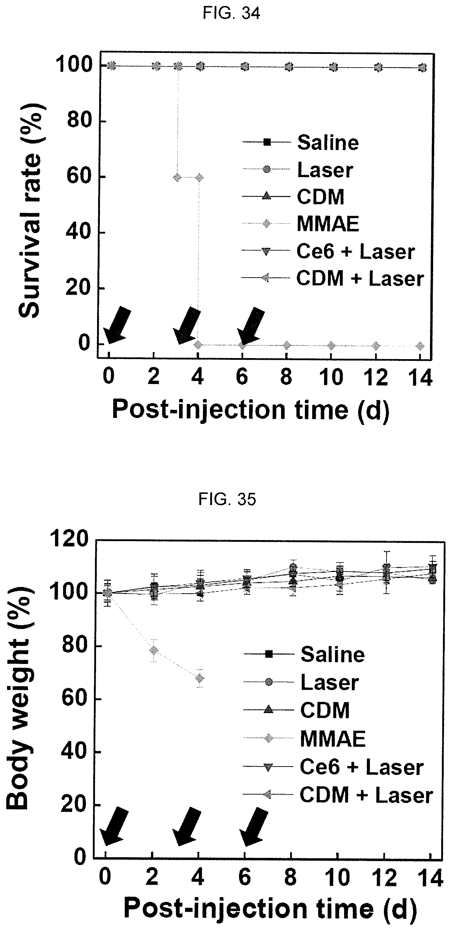

FIGS. 34-41 show the result of conducting experiments to evaluate the cytotoxicity of CDM in the animal model.

First, FIG. 34 shows a result of measuring the survival rate (%) of each group of the tumor animal model with time. As seen from FIG. 34, the cells of the MMAE group began to die from day 3 and all were killed on day 4. In contrast, the CDM, laser and Ce6 groups showed no toxicity at all in vivo, with a survival rate of 100%.

FIG. 35 shows a result of measuring the change in body weight (%) of each group of the tumor animal model with time. It can be seen that the body weight was decreased to 31.9 ± 1.6% only for the MMAE group. It may be due to the toxicity of MMAE.

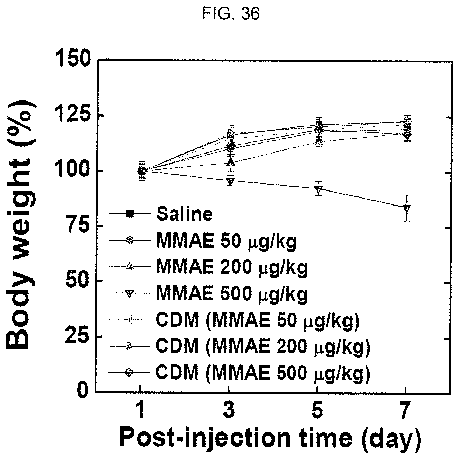

FIG. 36 shows a result of measuring the change in body weight with time for the tumor animal model to which MMAE (50, 200, 500 µg/kg) or CDM (50, 200, 500 µg/kg based on MMAE concentration) was administered. The change in body weight was hardly observed for the groups to which CDM was administered at different concentrations (4.8 ± 0.2%). In contrast, significant change in body weight was observed for the groups to which MMAE was administered at different concentrations (31.6 ± 2.2%).

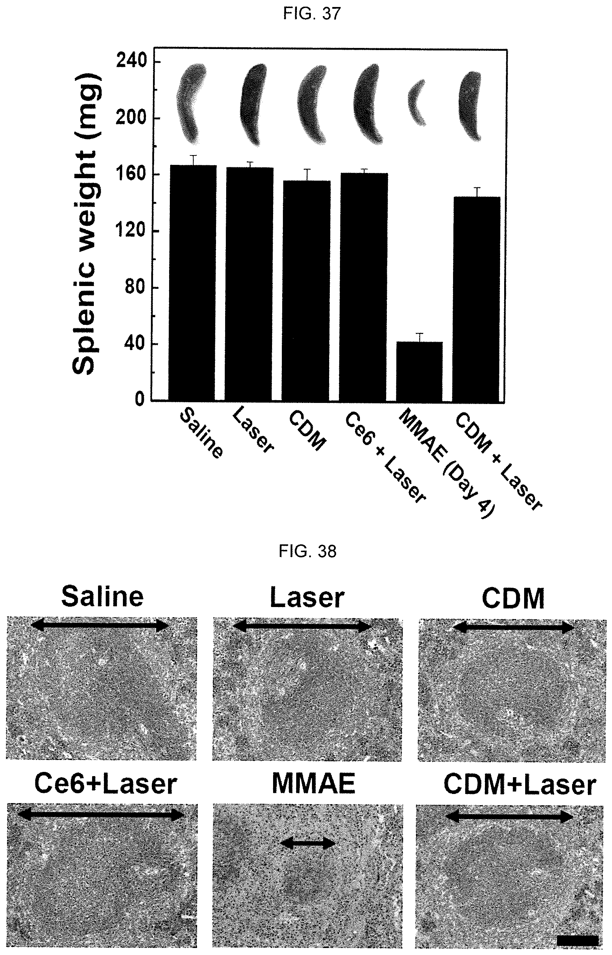

FIG. 37 shows a result of measuring the spleen weight (mg) of each group of the tumor animal model. It can be seen that the spleen weight was decreased to 75 ± 11.8% for the MMAE group only. The spleen could hardly function because of size reduction.

FIG. 38 shows a result of extracting the spleen from each group of the tumor animal model and analyzing the change in lymphocytes (lymphoid tissue; white pulp). In histological analysis, the oval white pulp corresponds to the lymphoid tissue. It was confirmed that the size of lymphocytes was significantly decreased for the MMAE group. This means that MMAE has immunotoxicity.

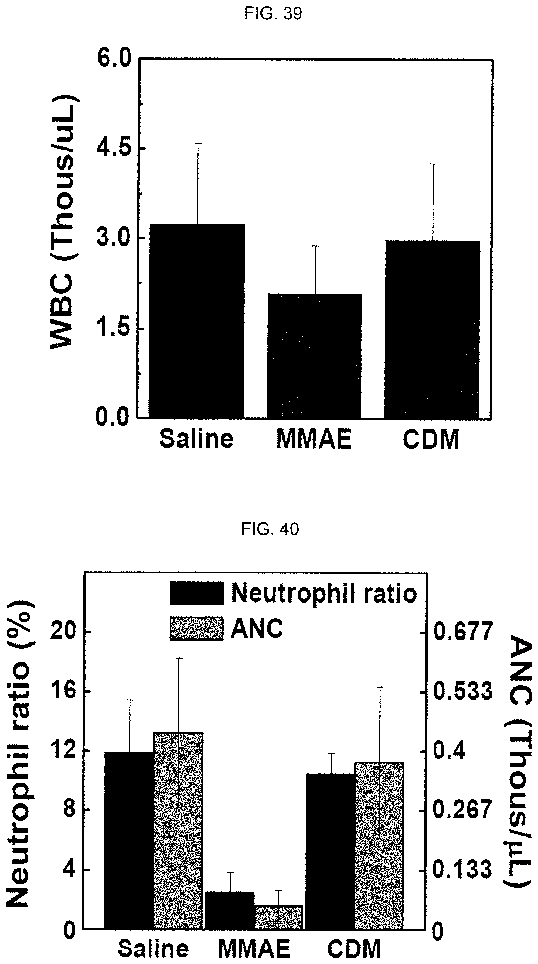

FIGS. 39-41 show a result of conducting blood toxicity test for each group. First, FIG. 39 shows a result of counting the number of total white blood cells (WBCs) in plasma for the CDM group (0.5 mg/kg based on MMAE concentration) and the MMAE group (0.5 mg/kg). It was confirmed that the CDM group shows no change in the number of total white blood cells (WBCs) because it forms a stable prodrug nanoparticle. In contrast, the MMAE group showed slightly decreased number of total white blood cells (35.7 ± 13.8%).

FIG. 40 shows a result of measuring the blood neutrophil ratio (%) for the CDM group (0.5 mg/kg based on MMAE concentration) and the MMAE group (0.5 mg/kg). It was confirmed that the MMAE group rapidly decreases the number of circulating neutrophils. That is to say, whereas the CDM group containing the same MMAE showed only slight decrease in the number of neutrophils (1.6 ± 1.0000/µL), the MMAE group showed 4.9 times less neutrophils as compared to the saline group. Specifically, the percentage of the neutrophils to the total white blood cells was decreased from 79.3% to 2.5 ± 1.4% for MMAE group.

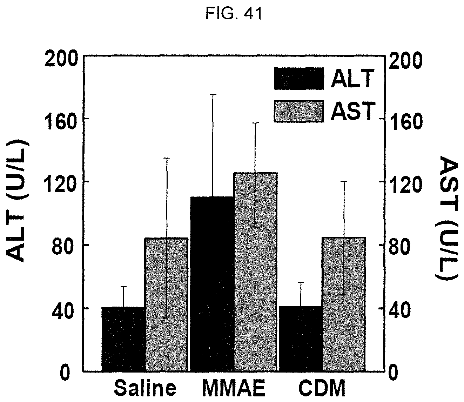

FIG. 41 shows a result of measuring the plasma level of liver enzymes including aspartate aminotransferase (AST) and alanine aminotransferase (ALT) for the CDM group (0.5 mg/kg based on MMAE concentration) and the MMAE group (0.5 mg/kg). It was confirmed that the MMAE group shows remarkably increased liver toxicity. Specifically, the MMAE group showed 2.7 times increased AST and 1.5 times increased ALT. To conclude these results, it can be seen that the CDM according to the present disclosure is a very effective therapeutic agent exhibiting few side effects in vivo.

### <Conclusion>

The existing nanoparticles are limited in clinical applications despite their superior function. It is because the anticancer therapy using nanocarriers has a therapeutic efficiency of less than 5%, exhibits toxicity for normal tissues, preparation processes are complicated or sensitive and the associated operation is difficult. The present disclosure was completed in an effort to solve the above-described problems and develop a therapeutic agent of a new structure which has specific activity for a tumor tissue and is safe and simple.

The photosensitizer-drug conjugate of a new structure according to the present disclosure is advantageous in that it has a tumor cell-specific targeting effect and can selectively induce apoptosis in response to light irradiation.

Because the photosensitizer-drug conjugate according to the present disclosure forms a nanoparticle in vivo through self-assembly, it has superior specific activity for a tumor tissue and is successfully accumulated in the tumor tissue. As the DEVD peptide is cleaved by caspase-3, the drug is released from the prodrug form and effectively acts on the tumor tissue.

In addition, because the photosensitizer-drug conjugate according to the present disclosure is in a prodrug form exhibiting no cytotoxicity at all and forms a nanoparticle through self-assembly, the problems of a complicated preparation process and use of an additional drug carrier can be solved. That is to say, the photosensitizer-drug conjugate of the present disclosure exists as a biocompatible material which is not limited in clinical use at normal times, unlike other existing nanoparticles.

Upon light irradiation, the photosensitizer-drug conjugate enhances oxidative stress as a PDT agent by generating ROS. And, in the presence of caspase-3, it exhibits the activity of quickly inducing apoptosis by releasing the anticancer agent MMAE. It is very effective in that photodynamic therapy and pharmaceutical therapeutic effect are exerted at the same time from one material.

In other words, the photosensitizer-drug conjugate according to the present disclosure exists as a nontoxic form despite the absence of an additional carrier at normal times and is specifically accumulated in a tumor tissue by the EPR effect and is converted from a prodrug form to an activated form in the tumor tissue. When light is irradiated to the tumor tissue, the photosensitizer-drug conjugate primarily induces generation of ROS, thereby inducing the apoptosis of nearby tumor cells. Secondarily, tumor cell death is induced by the anticancer agent released as the photosensitizer-drug conjugate is cleaved by caspase-3. Accordingly, the photosensitizer-drug conjugate exhibits very effective preventive or therapeutic effect for the tumor tissue and completely inhibits tumor growth even at low concentrations due to the continuous and lasting anticancer effect.

In addition, the photosensitizer-drug conjugate according to the present disclosure is advantageous in that it exhibits an extended blood circulation halflife and a remarkably decreased toxicity for normal tissues, such that it does not cause side effects for tissues other than those containing tumor cells, and is clinically applicable because the activation mechanism of the photosensitizerconjugate is achieved through a reliable process.

In cytotoxicity test, it was confirmed that the photosensitizer-drug conjugate according to the present disclosure exhibits little toxicity whereas the existing anticancer agent resulted in death of animals due to strong toxicity. Accordingly, the photosensitizer-drug conjugate according to the present disclosure has double stability because it exists as a prodrug nanoparticle form with no toxicity at all even when it is accumulated in the tumor cell until it is activated by light irradiation.

## Claims

1. A self-assembling tumor-targeting photosensitizer-drug conjugate wherein a photosensitizer, a peptide, a linker and an anticancer agent are conjugated sequentially, wherein the peptide is a peptide which is conjugated to one side of the photosensitizer and comprises a sequence that can be specifically cleaved by caspase, the linker is conjugated to one end of the peptide and connects the peptide with the anticancer agent,
and
wherein the photosensitizer-drug conjugate is represented by Structural Formula 1:

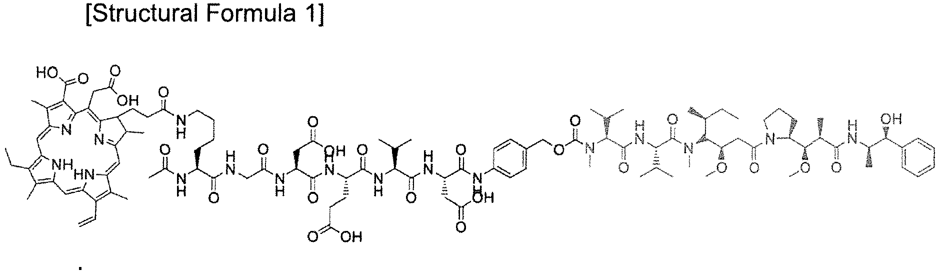

2. The tumor-targeting photosensitizer-drug conjugate according to claim 1, wherein the tumor-targeting photosensitizer-drug conjugate forms a nanoparticle structure in a solution through self-assembly.

3. A method for preparing a photosensitizer-drug conjugate, comprising:
a) in a peptide comprising a sequence that can be cleaved by caspase, substituting the hydrogen of amino acid residues excluding the site to which a linker is to be conjugated with an allyl group or an allyloxycarbonyl group;
b) conjugating a linker to the C-terminal of the substituted peptide, wherein the linker is one or more selected from a group consisting of a small number of carbons, a peptide, polyethylene glycol (PEG) and p-aminobenzyloxy carbamate (PABC),
c) preparing a drug conjugate by conjugating an anticancer agent to the linker;
d) deprotecting the substituted peptide of the drug conjugate prepared in
c) by substituting the allyl group or the allyloxycarbonyl group with hydrogen; and
e) conjugating a photosensitizer to the N-terminal amino group of the deprotected peptide.

4. The method for preparing a photosensitizer-drug conjugate according to claim 3, wherein the peptide comprising a sequence that can be cleaved by caspase in a) is represented by SEQ ID NO 1.

5. The method for preparing a photosensitizer-drug conjugate according to claim 3 or 4, wherein, in a) of preparing the substituted peptide, the carboxyl hydrogen of the side chain of the peptide comprising SEQ ID NO 1 that can be cleaved by caspase is substituted with the allyl group, the amino hydrogen of the side chain is substituted with the allyloxycarbonyl group, and the N-terminal amine group is protected with an acetyl group.

6. A pharmaceutical composition for use in preventing or treating a cancer, comprising the photosensitizer-drug conjugate according to any of the claims 1 to 2 as an active ingredient.

7. The pharmaceutical composition for use in preventing or treating a cancer according to claim 6, wherein the pharmaceutical composition comprising the photosensitizer-drug conjugate is selectively accumulated at a tumor site and induces selective death of a tumor cell when light is irradiated, the photosensitizer-drug conjugate is cleaved by caspase-3 existing in the tumor cell and an anticancer effect is exhibited as a drug is released from the photosensitizer-drug conjugate which is in a prodrug form.

8. The pharmaceutical composition for use in preventing or treating a cancer according to claim 6 or 7, wherein the cancer is one or more selected from a group consisting of brain tumor, benign astrocytoma, malignant astrocytoma, pituitary adenoma, brain lymphoma, oligodendroglioma, craniopharyngioma, ependymoma, brain stem tumor, head and neck tumor, laryngeal cancer, oropharyngeal cancer, nasal cavity/paranasal sinus cancer, nasopharyngeal cancer, salivary gland cancer, hyopphayngeal cancer, thyroid cancer, oral cancer, breast tumor, small-cell lung cancer, non-small-cell lung cancer, thymus cancer, mediastinal tumor, esophageal cancer, breast cancer, male breast cancer, abdominal tumor, stomach cancer, liver cancer, gallbladder cancer, bile duct cancer, pancreatic cancer, small intestine cancer, large intestine cancer, anal cancer, bladder cancer, renal cancer, prostate cancer, testicular cancer, uterine cancer, cervical cancer, endometrial cancer, ovarian cancer, uterine sarcoma, squamous cell carcinoma and skin cancer.

9. The pharmaceutical composition for use in preventing or treating a cancer according to any of the claims 6 to 8, wherein the pharmaceutical composition is injected by intravenous or topical administration.

## Patentansprüche

1. Selbstorganisierendes Photosensibilisator-Wirkstoff-Konjugat zur gezielten Krebstherapie, wobei ein Photosensibilisator, ein Peptid, ein Linker und ein Krebsmedikament sequenziell konjugiert sind, wobei es sich bei dem Peptid um ein Peptid handelt, das an eine Seite des Photosensibilisators konjugiert ist und eine Sequenz umfasst, die durch Caspase spezifisch spaltbar ist, der Linker an ein Ende des Peptids konjugiert ist und das Peptid mit dem Krebsmedikament verbindet und wobei das Photosensibilisator-Wirkstoff-Konjugat durch die Strukturformel 1 dargestellt ist:

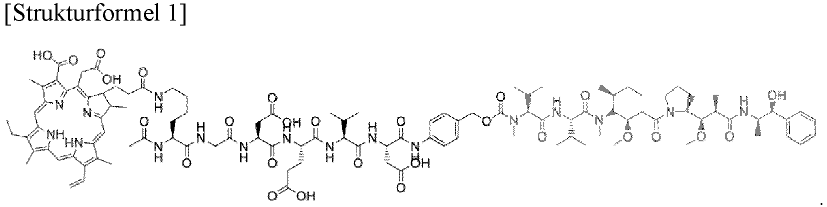

2. Photosensibilisator-Wirkstoff-Konjugat zur gezielten Krebstherapie nach Anspruch 1, wobei das Photosensibilisator-Wirkstoff-Konjugat zur gezielten Krebstherapie in einer Lösung durch Selbstorganisation eine Nanopartikelstruktur bildet.

3. Verfahren zur Herstellung eines Photosensibilisator-Wirkstoff-Konjugats, umfassend:
a) Substituieren des Wasserstoffs von Aminosäureresten bei einem eine durch Caspase spaltbare Sequenz umfassenden Peptid außer an der Stelle, an der ein Linker mit einer Allyl-Gruppe oder einer Allyloxycarbonyl-Gruppe konjugiert werden soll;
b) Konjugieren eines Linkers an den C-Terminus des substituierten Peptids, wobei es sich bei dem Linker wahlweise um eine kleine Anzahl von Kohlenstoffen, ein Peptid, Polyethylenglycol (PEG) und/oder p-Aminobenzyloxycarbamat (PABC) handelt,
c) Herstellen eines Wirkstoff-Konjugats durch Konjugieren eines Krebsmedikaments an den Linker;
d) Entschützen des substituierten Peptids des in Schritt c) hergestellten Wirkstoff-Konjugats durch Substituieren der Allyl-Gruppe oder der Allyloxycarbonyl-Gruppe durch Wasserstoff; und
e) Konjugieren eines Photosensibilisators an die N-terminale Aminogruppe des entschützten Peptids.

4. Verfahren zur Herstellung eines Photosensibilisator-Wirkstoff-Konjugats nach Anspruch 3, wobei das eine in Schritt a) durch Caspase spaltbare Sequenz umfassende Peptid durch SEQ ID NO 1 dargestellt ist.

5. Verfahren zur Herstellung eines Photosensibilisator-Wirkstoff-Konjugats nach Anspruch 3 oder 4, wobei in Schritt a) des Herstellens des substituierten Peptids der Carboxyl-Wasserstoff der Seitenkette des die durch Caspase spaltbare SEQ ID NO 1 umfassenden Peptids durch die Allyl-Gruppe substituiert wird, der Amino-Wasserstoff der Seitenkette durch die Allyloxycarbonyl-Gruppe substituiert wird und die N-terminale Amin-Gruppe mit einer Acetyl-Gruppe geschützt wird.

6. Pharmazeutische Zusammensetzung zur Verwendung bei der Vorbeugung oder Behandlung eines Krebses, umfassend das Photosensibilisator-Wirkstoff-Konjugat nach einem der Ansprüche 1 bis 2 als aktiven Bestandteil.

7. Pharmazeutische Zusammensetzung zur Verwendung bei der Vorbeugung oder Behandlung eines Krebses nach Anspruch 6, wobei die das Photosensibilisator-Wirkstoff-Konjugat umfassende pharmazeutische Zusammensetzung sich selektiv an einem Tumor ansammelt und bei Bestrahlung mit Licht den selektiven Zelltod einer Tumorzelle induziert, wobei das Photosensibilisator-Wirkstoff-Konjugat durch in der Tumorzelle vorhandene Caspase-3 gespalten wird und sich eine Antikrebswirkung entfaltet, indem ein Wirkstoff aus dem Photosensibilisator-Wirkstoff-Konjugat freigesetzt wird, der in Form einer Prodrug vorliegt.

8. Pharmazeutische Zusammensetzung zur Verwendung bei der Vorbeugung oder Behandlung eines Krebses nach Anspruch 6 oder 7, wobei es sich bei dem Krebs wahlweise um einen Gehirntumor, ein benignes Astrozytom, ein malignes Astrozytom, ein Hypophysenadenom, ein Lymphom des Gehirns, ein Oligodendrogliom, ein Kraniopharyngeom, ein Ependymom, einen Hirnstammtumor, einen Kopf-Hals-Tumor, Kehlkopfkrebs, ein Oropharynxkarzinom, Nasennebenhöhlenkrebs, ein Nasopharynxkarzinom, Speicheldrüsenkrebs, ein Hypopharynxkarzinom, Schilddrüsenkrebs, Mundhöhlenkrebs, Brustkrebs, ein kleinzelliges Lungenkarzinom, ein nicht-kleinzelliges Lungenkarzinom, ein Thymuskarzinom, einen Mediastinaltumor, Speiseröhrenkrebs, Brustkrebs, Brustkrebs beim Mann, einen Abdominaltumor, Magenkrebs, Leberkrebs, Gallenblasenkrebs, Gallengangkrebs, Bauchspeicheldrüsenkrebs, Dünndarmkrebs, Dickdarmkrebs, ein Analkarzinom, Blasenkrebs, Nierenkrebs, Prostatakrebs, Hodenkrebs, Gebärmutterkrebs, Gebärmutterhalskrebs, ein Endometriumkarzinom, ein Ovarialkarzinom, ein Sarkom der Gebärmutter, ein Plattenepithelkarzinom und/oder Hautkrebs handelt.

9. Pharmazeutische Zusammensetzung zur Verwendung bei der Vorbeugung oder Behandlung eines Krebses nach einem der Ansprüche 6 bis 8, wobei die pharmazeutische Zusammensetzung intravenös injiziert oder topisch verabreicht wird.

## Revendications

1. Conjugué médicament-photosensibilisateur auto-assemblant ciblant des tumeurs, dans lequel un photosensibilisateur, un peptide, un lieur et un agent anticancéreux sont conjugués séquentiellement, dans lequel le peptide est un peptide qui est conjugué à un côté du photosensibilisateur et comprend une séquence qui peut être clivée spécifiquement par la caspase, le lieur est conjugué à une extrémité du peptide and relie le peptide et l'agent anticancéreux et
dans lequel le conjugué médicament-photosensibilisateur est représenté par la Formule Structurelle 1 :

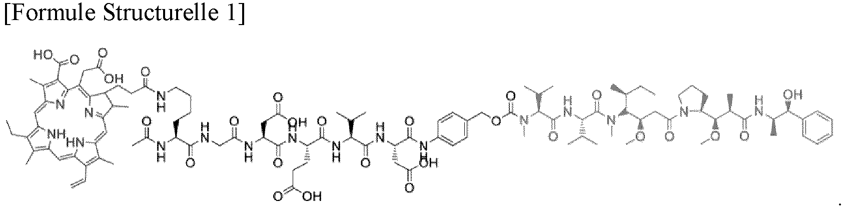

2. Conjugué médicament-photosensibilisateur ciblant des tumeurs selon la revendication 1, dans lequel le conjugué médicament-photosensibilisateur ciblant des tumeurs forme une structure nano-particulaire dans une solution par auto-assemblage.

3. Procédé de préparation d'un conjugué médicament-photosensibilisateur, le procédé comprenant les étapes consistant à :
a) dans un peptide comprenant une séquence qui peut être clivée par la caspase, substituer l'hydrogène de résidus d'acide amine sauf la site à laquelle un lieur doit être conjugué avec un groupe allyle ou un groupe allyloxycarbonyle ;
b) conjuguer un lieur à l'extrémité C-terminale du peptide substitué, dans lequel le lieur est un ou plusieurs sélectionnés dans un groupe consistant à un petit nombre de carbones, un peptide, polyéthylène glycol (PEG) et p-aminobenzyloxy carbamate (PABC),
c) préparer un conjugué médicament an conjuguant un agent anticancéreux au lieur ;
d) déprotéger le peptide substitué du conjugué médicament préparé dans l'étape c) en substituant le groupe allyle ou le groupe allyloxycarbonyle à l'hydrogène ; et
e) conjuguer un photosensibilisateur au groupe amino N-terminal du peptide déprotégé.

4. Procédé de préparation d'un conjugué médicament-photosensibilisateur selon la revendication 3, dans lequel le peptide comprenant une séquence qui peut être clivée par la caspase dans l'étape a) est représenté par SEQ ID NO 1.

5. Procédé de préparation d'un conjugué médicament-photosensibilisateur selon la revendication 3 ou 4, dans lequel, dans l'étape a) consistant à préparer le peptide substitué, l'hydrogène de carboxyle de la chaîne latérale du peptide comprenant la SEQ ID NO 1 qui peut être clivée par la caspase est substitué au groupe allyle, l'hydrogène d'amino de la chaîne latérale est substitué au groupe allyloxycarbonyl et le groupe amine N-terminal est protégé par un groupe acétyle.

6. Composition pharmaceutique pour l'utilisation dans la prévention ou le traitement d'un cancer, la composition comprenant le conjugué médicament-photosensibilisateur selon l'une quelconque des revendications 1 à 2 comme ingrédient actif.

7. Composition pharmaceutique pour l'utilisation dans la prévention ou le traitement d'un cancer selon la revendication 6, dans laquelle la composition pharmaceutique comprenant le conjugué médicament-photosensibilisateur s'accumule sélectivement au niveau d'un site tumoral et induit la mort sélective d'une cellule tumorale sous irradiation par de la lumière, le conjugué médicament-photosensibilisateur étant clivé par la caspase-3 présente dans la cellule tumorale et un effet anticancéreux est exercé par la libération d'un médicament du conjugué médicament-photosensibilisateur qui se présente sous forme de promédicament.

8. Composition pharmaceutique pour l'utilisation dans la prévention ou le traitement d'un cancer selon la revendication 6 ou 7, dans laquelle le cancer est un ou plusieurs choisis parmi un groupe constitué d'une tumeur cérébrale, d'un astrocytome bénin, d'un astrocytome malin, d'un adénome hypophysaire, d'un lymphome cérébral, d'un oligodendrogliome, d'un craniopharyngiome, d'un épendymome, d'une tumeur du tronc cérébral, d'un tumeur de la tête et du cou, d'un cancer du larynx, d'un cancer de l'oropharynx, d'un cancer de la cavité nasale/des sinus paranasaux, d'un cancer du nasopharynx, d'un cancer des glandes salivaires, d'un cancer de l'hypopharynx, d'un cancer de la thyroïde, d'un cancer de la bouche, d'une tumeur du sein, d'un cancer du poumon à petites cellules, d'un cancer du poumon non à petites cellules, d'un cancer du thymus, d'une tumeur médiastinale, d'un cancer de l'œsophage, d'un cancer du sein, d'un cancer du sein chez l'homme, d'une tumeur abdominale, d'un cancer de l'estomac, d'un cancer du foie, d'un cancer de la vésicule biliaire, d'un cancer des voies biliaires, d'un cancer du pancréas, d'un cancer de l'intestin grêle, d'un cancer du gros intestin, d'un cancer de l'anus, d'un cancer de la vessie, d'un cancer du rein, d'un cancer de la prostate, d'un cancer des testicules, d'un cancer de l'utérus, d'un cancer du col de l'utérus, d'un cancer de l'endomètre, d'un cancer de l'ovaire, d'un sarcome utérin, d'un carcinome épidermoïde et d'un cancer de la peau.

9. Composition pharmaceutique pour l'utilisation dans la prévention ou le traitement d'un cancer selon l'une quelconque des revendications 6 à 8, dans laquelle la composition pharmaceutique est administrée par injection intraveineuse ou par voie topique.
